# EUROPEAN PATENT SPECIFICATION

(11) **EP 1 570 062 B1**
(45) Date of publication and mention of the grant of the patent: **15.03.2006**
(21) Application number: 03812594.4
(22) Date of filing: 09.12.2003
(51) Int. Cl.: C12N 15/67

(54) **OPTIMISED PROTEIN SYNTHESIS**
OPTIMIERTE PROTEINSYNTHESE
SYNTHESE PROTEIQUE OPTIMISEE

(30) Priority: 09.12.2002 DE 10257479
(43) Date of publication of application: 07.09.2005
(73) Proprietor: F. HOFFMANN-LA ROCHE AG, 4070 Basel (CH); Roche Diagnostics GmbH, 68305 Mannheim (DE)
(72) Inventor: WATZELE, Manfred, 82362 Weilheim (DE); BUCHBERGER, Bernd, 82380 Peissenberg (DE); PAULUS, Michael, 86199 Augsburg (DE)
(74) Representative: Weiss, Wolfgang
(86) International application number: PCT/EP2003/013964
(87) International publication number: WO 2004/053053

(56) References cited:
- WO-A-89/00604
- STENSTROM C MAGNUS ET AL: "Cooperative effects by the initiation codon and its flanking regions on translation initiation" GENE (AMSTERDAM), Bd. 273, Nr. 2, 8. August 2001 (2001-08-08), Seiten 259-265, XP002285920 ISSN: 0378-1119
- SPANJAARD R A ET AL: "EXPRESSION OF THE RAT INTERFERON-ALPHA-1 GENE IN ESCHERICHIA-COLI CONTROLLED BY THE SECONDARY STRUCTURE OF THE TRANSLATION-INITIATION REGION" GENE, ELSEVIER BIOMEDICAL PRESS. AMSTERDAM, NL, Bd. 80, Nr. 2, 1989, Seiten 345-351, XP002182303 ISSN: 0378-1119

### SEQUENCE LISTING

<110> Roche Diagnostics GmbH
<120> Optimierte Proteinsynthese
<130> 29415pwo
<140> PCT/EP03
   <141> 2003-12-09
<160> 57
<170> Patent In Ver. 2.1
<210> 1
   <211> 84
   <212> DNA
   <213> Artificial Sequence
<220>
   <223> Description of Artificial Sequence:Primer C
<400> 1
   gaaattaata cgactcacta tagggagacc acaacggttt ccctctagaa ataattttgt 60
   ttaactttaa gaaggagata tacc                                        84
<210> 2
   <211> 71
   <212> DNA
   <213> Artificial Sequence
<220>
   <223> Description of Artificial Sequence:Primer D
<400> 2
   caaaaaaccc ctcaagaccc gtttagaggc cccaaggggg gccgccagtg tgctgaattc 60
   gccttttatt a 71
<210> 3
   <211> 30
   <212> DNA
   <213> Artificial Sequence
<220>
   <223> Description of Artificial Sequence:Primer A without hairpinloop
<400> 3
   aggagatata ccatgactag caaaggagaa 30
<210> 4
   <211> 42
   <212> DNA
   <213> Artificial Sequence
<220>
   <223> Description of Artificial Sequence:Primer A Stammlänge 4 bp
<400> 4
   aggagatata ccatgactaa ttttagtact agcaaaggag aa 42
<210> 5
   <211> 45
   <212> DNA
   <213> Artificial Sequence
<220>
   <223> Description of Artificial Sequence:Primer A Stammlänge 5 bp
<400> 5
   aggagatata ccatgactgt ttatacagta actagcaaag gagaa 45
<210> 6
   <211> 48
   <212> DNA
   <213> Artificial Sequence
<220>
   <223> Description of Artificial Sequence:Primer A Stammiänge 6 bp
<400> 6
   aggagatata ccatgactgg tcaattacca gtaactagca aaggagaa 48
<210> 7
   <211> 51
   <212> DNA
<213> Artificial Sequence
<220>
   <223> Description of Artificial Sequence:Primer A Stammlänge 7 bp
<400> 7
   aggagatata ccatgactgc tttacatcaa gcagtaacta gcaaaggaga a 51
<210> 8
   <211> 51
   <212> DNA
   <213> Artificial Sequence
<220>
   <223> Description of Artificial Sequence:Primer A Stammlänge 8 bp
<400> 8
   aggagatata ccatgactgc acgtgatcgt gcagtaacta gcaaaggaga a 51
<210> 9
   <211> 30
   <212> DNA
   <213> Artificial Sequence
<220>
   <223> Description of Artificial Sequence:Primer B
<400> 9
   attcgccttt tattaatgat gatgatgatg 30
<210> 10
   <211> 60
   <212> DNA
   <213> Artificial Sequence
<220>
   <223> Description of Artificial Sequence:Primer A
<400> 10
   aggagatata ccatgactag cactgcacgt gcatcgtgca gtgtaaaagg agaagaactt 60
<210> 11
   <211> 63
   <212> DNA
   <213> Artificial Sequence
<220>
   <223> Description of Artificial Sequence:Primer A
<400> 11
   aggagatata ccatgactag caaaactgca cgtgcatcgt gcagtgtagg agaagaactt 60
   ttc 63
<210> 12
   <211> 66
   < 212 > DNA
   <213> Artificial Sequence
<220>
   <223> Description of Artificial Sequence:Primer A
<400> 12
   aggagatata ccatgactag caaaggaact gcacgtgcat cgtgcagtgt agaagaactt 60
   ttcact 66
<210> 13
   <211> 69
   <212> DNA
   <213> Artificial Sequence
<220>
   <223> Description of Artificial Sequence:Primer A
<400> 13
   aggagatata ccatgactag caaaggagaa actgcacgtg catcgtgcag tgtagaactt 60
   ttcactgga 69
<210> 14
   <211> 72
   <212> DNA
   <213> Artificial Sequence
<220>
   <223> Description of Artificial Sequence:Primer A
<400> 14
   aggagatata ccatgactag caaaggagaa gaaactgcac gtgcatcgtg cagtgtactt 60
   ttcactggag tt 72
<210> 15
   <211> 75
   <212> DNA
   <213> Artificial Sequence
<220>
   <223> Description of Artificial Sequence:Primer A
<400> 15
   aggagatata ccatgactag caaaggagaa gaacttactg cacgtgcatc gtgcagtgta 60
   ttcactggag ttgtc 75
<210> 16
   <211> 71
   <212> DNA
   <213> Artificial Sequence
<220>
   <223> Description of Artificial Sequence:Primer D
<400> 16
   caaaaaaccc ctcaagaccc gtttagaggc cccaaggggt tgggagtaga atgttaagga 60
   ttagtttatt a 71
<210> 17
   <211> 60
   <212> DNA
   <213> Artificial Sequence
<220>
   <223> Description of Artificial Sequence:Primer A
<400> 17
   aggagatata ccatgaaata tacatattct ctgcacgtga tcgtgcaggc taacaccgcg 60
<210> 18
   <211> 60
   <212> DNA
   <213> Artificial Sequence
<220>
   <223> Description of Artificial Sequence:Primer A
<400> 18
   aggagatata ccatgaaaac atattattct ctgcacgtga tcgtgcaggc taacaccgcg 60
<210> 19
   <211> 60
   <212> DNA
   <213> Artificial Sequence
<220>
   <223> Description of Artificial Sequence:Primer A
<400> 19
   aggagatata ccatgaaata ttcttataca ctgcacgtga tcgtgcaggc taacaccgcg 60
<210> 20
   <211> 60
   <212> DNA
   <213> Artificial Sequence
<220>
   <223> Description of Artificial Sequence:Primer A
<400> 20
   aggagatata ccatgaaata ttattctaca ctgcacgtga tcgtgcaggc taacaccgcg 60
<210> 21
   <211> 60
   <212> DNA
   <213> Artificial Sequence
<220>
   <223> Description of Artificial Sequence:Primer A
<400> 21
   aggagatata ccatgaaata tacatattca ctgcacgtga tcgtgcaggc taacaccgcg 60
<210> 22
   <211> 60
   <212> DNA
   <213> Artificial Sequence
<220>
   <223> Description of Artificial Sequence:Primer A
<400> 22
   aggagatata ccatgaaaac atattattca ctgcacgtga tcgtgcaggc taacaccgcg 60
<210> 23
   <211> 60
   <212> DNA
   <213> Artificial Sequence
<220>
   <223> Description of Artificial Sequence:Primer A
<400> 23
   aggagatata ccatgaaata ttcatataca ctgcacgtga tcgtgcaggc taacaccgcg 60
<210> 24
   <211> 60
   <212> DNA
   <213> Artificial Sequence
<220>
   <223> Description of Artificial Sequence:Primer A
<400> 24
   aggagatata ccatgaaata ttattcaaca ctgcacgtga tcgtgcaggc taacaccgcg 60
<210> 25
   <211> 60
   <212> DNA
   <213> Artificial Sequence
<220>
   <223> Description of Artificial Sequence:Primer A
<400> 25
   aggagatata ccatgcatca tcatcatcat ctgcacgtga tcgtgcaggc taacaccgcg 60
<210> 26
   <211> 27
   <212> DNA
   <213> Artificial Sequence
<220>
   <223> Description of Artificial Sequence:Primer Wildtyp
<400> 26
   aggagatata ccatggctaa caccgcg 27
<210> 27
   <211> 48
   <212> DNA
   <213> Artificial Sequence
<220>
   <223> Description of Artificial Sequence:Primer B
<400> 27
   aggattagtt tattaatgat gatgatgatg atggcgccgg gtgcgcga 48
<210> 28
   <211> 60
   <212> DNA
   <213> Artificial Sequence
<220>
   <223> Description of Artificial Sequence:Primer A
<400> 28
   aggagatata ccatgaaata tacatattct ctgcacgtga tcgtgcaggg tgccccgacg 60
<210> 29
   <211> 60
   <212> DNA
   <213> Artificial Sequence
<220>
   <223> Description of Artificial Sequence:Primer A
<400> 29
   aggagatata ccatgaaaac atattattct ctgcacgtga tcgtgcaggg tgccccgacg 60
<210> 30
   <211> 60
   <212> DNA
   <213> Artificial Sequence
<220>
   <223> Description of Artificial Sequence:Primer A
<400> 30
   aggagatata ccatgaaata ttcttataca ctgcacgtga tcgtgcaggg tgccccgacg 60
<210> 31
   <211> 60
   <212> DNA
   <213> Artificial Sequence
<220>
   <223> Description of Artificial Sequence:Primer A
<400> 31
   aggagatata ccatgaaata ttattctaca ctgcacgtga tcgtgcaggg tgccccgacg 60
<210> 32
   <211> 60
   <212> DNA
   <213> Artificial Sequence
<220>
   <223> Description of Artificial Sequence:Primer A
<400> 32
   aggagatata ccatgaaata tacatattca ctgcacgtga tcgtgcaggg tgccccgacg 60
<210> 33
   <211> 60
   <212> DNA
   <213> Artificial Sequence
<220>
   <223> Description of Artificial Sequence:Primer A
<400> 33
   aggagatata ccatgaaaac atattattca ctgcacgtga tcgtgcaggg tgccccgacg 60
<210> 34
   <211> 60
   <212> DNA
   <213> Artificial Sequence
<220>
   <223> Description of Artificial Sequence:Primer A
<400> 34
   aggagatata ccatgaaata ttcatataca ctgcacgtga tcgtgcaggg tgccccgacg 60
<210> 35
   <211> 60
   <212> DNA
   <213> Artificial Sequence
<220>
   <223> Description of Artificial Sequence:Primer A
<400> 35
   aggagatata ccatgaaata ttattcaaca ctgcacgtga tcgtgcaggg tgccccgacg 60
<210> 36
   <211> 60
   <212> DNA
   <213> Artificial Sequence
<220>
   <223> Description of Artificial Sequence:Primer A
<400> 36
   aggagatata ccatgcatca tcatcatcat ctgcacgtga tcgtgcaggg tgccccgacg 60
<210> 37
   <211> 27
   <212> DNA
   <213> Artificial Sequence
<220>
   <223> Description of Artificial Sequence:Primer A Wildtyp
<400> 37
   aggagatata ccatgggtgc cccgacg 27
<210> 38
   <211> 49
   <212> DNA
   <213> Artificial Sequence
<220>
   <223> Description of Artificial Sequence:Primer B
<400> 38
   aggattagtt tattaatgat gatgatgatg atgatccatg gcagccagc 49
<210> 39
   <211> 60
   <212> DNA
   <213> Artificial Sequence
<220>
   <223> Description of Artificial Sequence:Primer
<400> 39
   aggagatata ccatgaaata tacatattct ctgcacgtga tcgtgcagga gttggggccc 60
<210> 40
   <211> 60
   <212> DNA
   <213> Artificial Sequence
<220>
   <223> Description of Artificial Sequence:Primer
<400> 40
   aggagatata ccatgaaaac atattattct ctgcacgtga tcgtgcagga gttggggccc 60
<210> 41
   <211> 60
   <212> DNA
   <213> Artificial Sequence
<220>
   <223> Description of Artificial Sequence:Primer
<400> 41
   aggagatata ccatgaaata ttcttataca ctgcacgtga tcgtgcagga gttggggccc 60
<210> 42
   <211> 60
   <212> DNA
   <213> Artificial Sequence
<220>
   <223> Description of Artificial Sequence:Primer
<400> 42
   aggagatata ccatgaaata ttattctaca ctgcacgtga tcgtgcagga gttggggccc 60
<210> 43
   <211> 60
   <212> DNA
   <213> Artificial Sequence
<220>
   <223> Description of Artificial Sequence:Primer
<400> 43
   aggagatata ccatgaaata tacatattca ctgcacgtga tcgtgcagga gttggggccc 60
<210> 44
   <211> 60
   <212> DNA
   <213> Artificial Sequence
<220>
   <223> Description of Artificial Sequence:Primer
<400> 44
   aggagatata ccatgaaaac atattattca ctgcacgtga tcgtgcagga gttggggccc 60
<210> 45
   <211> 60
   <212> DNA
   <213> Artificial Sequence
<220>
   <223> Description of Artificial Sequence:Primer
<400> 45
   aggagatata ccatgaaata ttcatataca ctgcacgtga tcgtgcagga gttggggccc 60
<210> 46
   <211> 60
   <212> DNA
   <213> Artificial Sequence
<220>
   <223> Description of Artificial Sequence:Primer
<400> 46
   aggagatata ccatgaaata ttattcaaca ctgcacgtga tcgtgcagga gttggggccc 60
<210> 47
   <211> 60
   <212> DNA
   <213> Artificial Sequence
<220>
   <223> Description of Artificial Sequence:Primer
<400> 47
   aggagatata ccatgcatca tcatcatcat ctgcacgtga tcgtgcagga gttggggccc 60
<210> 48
   <211> 27
   <212> DNA
   <213> Artificial Sequence
<220>
   <223> Description of Artificial Sequence:Primer A Wildtyp
<400> 48
   aggagatata ccatggagtt ggggccc 27
<210> 49
   <211> 45
   <212> DNA
   <213> Artificial Sequence
<220>
   <223> Description of Artificial Sequence:Primer B
<400> 49
   aggattagtt tattattaat gatgatgatg atgatgagaa ccccc 45
<210> 50
   <211> 431
   <212> DNA
   <213> Artificial Sequence
<220>
   <223> Description of Artificial Sequence: Expressionskonstrukt für Mutante 1
<400> 50
   gaaattaata cgactcacta tagggagacc acaacggttt ccctctagaa ataattttgt 60
   ttaactttaa gaaggagata taccatgaaa tatacatatt ctctgcacgt gatcgtgcag 120
   gctaacaccg cgccgggacc cacggtggcc aacaagcggg acgaaaaaca ccgtcacgtc 180
   gttaacgtcg ttttggagct gccgaccgag atatcagagg ccacccaccc ggtgttggcc 240
   accatgctga gcaagtacac gcgcatgtcc agcctgttta atgacaagtg cgcctttaag 300
   ctggacctgt tgcgcatggt agccgtgtcg cgcacccggc gccatcatca tcatcatcat 360
   taataaacta atccttaaca ttctactccc aaccccttgg ggcctctaaa cgggtcttga 420
   ggggtttttt g 431
<210> 51
   <211> 398
   <212> DNA
   <213> Artificial Sequence
<220>
   <223> Description of Artificial Sequence: Expressionskonstrukt für Wildtyp
<400> 51
   gaaattaata cgactcacta tagggagacc acaacggttt ccctctagaa ataattttgt 60
   ttaactttaa gaaggagata taccatggct aacaccgcgc cgggacccac ggtggccaac 120
   aagcgggacg aaaaacaccg tcacgtcgtt aacgtcgttt tggagctgcc gaccgagata 180
   tcagaggcca cccacccggt gttggccacc atgctgagca agtacacgcg catgtccagc 240
   ctgtttaatg acaagtgcgc ctttaagctg gacctgttgc gcatggtagc cgtgtcgcgc 300
   acccggcgcc atcatcatca tcatcattaa taaactaatc cttaacattc tactcccaac 360
   cccttggggc ctctaaacgg gtcttgaggg gttttttg 398
<210> 52
   <211> 632
   <212> DNA
   <213> Artificial Sequence
<220>
   <223> Description of Artificial Sequence: Expresionskonstrukt Mutante 1
<400> 52
   gaaattaata cgactcacta tagggagacc acaacggttt ccctctagaa ataattttgt 60
   ttaactttaa gaaggagata taccatgaaa tatacatatt ctctgcacgt gatcgtgcag 120
   ggtgccccga cgttgccccc tgcctggcag ccctttctca aggaccaccg catctctaca 180
   ttcaagaact ggcccttctt ggagggctgc gcctgcaccc cggagcggat ggccgaggct 240
   ggcttcatcc actgccccac tgagaacgag ccagacttgg cccagtgttt cttctgcttc 300
   aaggagctgg aaggctggga gccagatgac gaccccatag aggaacataa aaagcattcg 360
   tccggttgcg ctttcctttc tgtcaagaag cagtttgaag aattaaccct tggtgaattt 420
   ttgaaactgg acagagaaag agccaagaac aaaattgcaa aggaaaccaa caataagaag 480
   aaagaatttg aggaaactgc gaagaaagtg cgccgtgcca tcgagcagct ggctgccatg 540
   gatcatcatc atcatcatca ttaataaact aatccttaac attctactcc caaccccttg 600
   gggcctctaa acgggtcttg aggggttttt tg                              632
<210> 53
   <211> 599
   <212> DNA
   <213> Artificial Sequence
<220>
   <223> Description of Artificial Sequence: Expressionskonstrukt Wildtyp
<400> 53
   gaaattaata cgactcacta tagggagacc acaacggttt ccctctagaa ataattttgt 60
   ttaactttaa gaaggagata taccatgggt gccccgacgt tgccccctgc ctggcagccc 120
   tttctcaagg accaccgcat ctctacattc aagaactggc ccttcttgga gggctgcgcc 180
   tgcaccccgg agcggatggc cgaggctggc ttcatccact gccccactga gaacgagcca 240
   gacttggccc agtgtttctt ctgcttcaag gagctggaag gctgggagcc agatgacgac 300
   cccatagagg aacataaaaa gcattcgtcc ggttgcgctt tcctttctgt caagaagcag 360
   tttgaagaat taacccttgg tgaatttttg aaactggaca gagaaagagc caagaacaaa 420
   attgcaaagg aaaccaacaa taagaagaaa gaatttgagg aaactgcgaa gaaagtgcgc 480
   cgtgccatcg agcagctggc tgccatggat catcatcatc atcatcatta ataaactaat 540
   ccttaacatt ctactcccaa ccccttgggg cctctaaacg ggtcttgagg ggttttttg  599
<210> 54
   <211> 1400
   <212> DNA
   <213> Artificial Sequence
<220>
   <223> Description of Artificial Sequence: Expressionskonstrukt Mutante 1
<400> 54
   gaaattaata cgactcacta tagggagacc acaacggttt ccctctagaa ataattttgt 60
   ttaactttaa gaaggagata taccatgaaa tatacatatt ctctgcacgt gatcgtgcag 120
   gagttggggc ccctagaagg tggctacctg gagcttctta acagcgatgc tgaccccctg 180
   tgcctctacc acttctatga ccagatggac ctggctggag aagaagagat tgagctctac 240
   tcagaacccg acacagacac catcaactgc gaccagttca gcaggctgtt gtgtgacatg 300
   gaaggtgatg aagagaccag ggaggcttat gccaatatcg cggaactgga ccagtatgtc 360
   ttccaggact cccagctgga gggcctgagc aaggacattt tcaagcacat aggaccagat 420
   gaagtgatcg gtgagagtat ggagatgcca gcagaagttg ggcagaaaag tcagaaaaga 480
   cccttcccag aggagcttcc ggcagacctg aagcactgga agccagctga gccccccact 540
   gtggtgactg gcagtctcct agtgggacca gtgagcgact gctccaccct gccctgcctg 600
   ccactgcctg cgctgttcaa ccaggagcca gcctccggcc agatgcgcct ggagaaaacc 660
   gaccagattc ccatgccttt ctccagttcc tcgttgagct gcctgaatct ccctgaggga 720
   cccatccagt ttgtccccac catctccact ctgccccatg ggctctggca aatctctgag 780
   gctggaacag gggtctccag tatattcatc taccatggtg aggtgcccca ggccagccaa 840
   gtaccccctc ccagtggatt cactgtccac ggcctcccaa catctccaga ccggccaggc 900
   tccaccagcc ccttcgctcc atcagccact gacctgccca gcatgcctga acctgccctg 960
   acctcccgag caaacatgac agagcacaag acgtccccca cccaatgccc ggcagctgga 1020
   gaggtctcca acaagcttcc aaaatggcct gagccggtgg agcagttcta ccgctcactg 1080
   caggacacgt atggtgccga gcccgcaggc ccggatggca tcctagtgga ggtggatctg 1140
   gtgcaggcca ggctggagag gagcagcagc aagagcctgg agcgggaact ggccaccccg 1200
   gactgggcag aacggcagct ggcccaagga ggcctggctg aggtgctgtt ggctgccaag 1260
   gagcaccggc ggccgcgtcg actcgagcga gctcccgggg ggggttctca tcatcatcat 1320
   catcattaat aataaactaa tccttaacat tctactccca accccttggg gcctctaaac 1380
   gggtcttgag gggttttttg                                            1400
<210> 55
   <211> 1367
   <212> DNA
   <213> Artificial Sequence
<220>
   <223> Description of Artificial Sequence: Expressionskonstrukt Wildtyp
<400> 55
   gaaattaata cgactcacta tagggagacc acaacggttt ccctctagaa ataattttgt 60
   ttaactttaa gaaggagata taccatggag ttggggcccc tagaaggtgg ctacctggag 120
   cttcttaaca gcgatgctga ccccctgtgc ctctaccact tctatgacca gatggacctg 180
   gctggagaag aagagattga gctctactca gaacccgaca cagacaccat caactgcgac 240
   cagttcagca ggctgttgtg tgacatggaa ggtgatgaag agaccaggga ggcttatgcc 300
   aatatcgcgg aactggacca gtatgtcttc caggactccc agctggaggg cctgagcaag 360
   gacattttca agcacatagg accagatgaa gtgatcggtg agagtatgga gatgccagca 420
   gaagttgggc agaaaagtca gaaaagaccc ttcccagagg agcttccggc agacctgaag 480
   cactggaagc cagctgagcc ccccactgtg gtgactggca gtctcctagt gggaccagtg 540
   agcgactgct ccaccctgcc ctgcctgcca ctgcctgcgc tgttcaacca ggagccagcc 600
   tccggccaga tgcgcctgga gaaaaccgac cagattccca tgcctttctc cagttcctcg 660
   ttgagctgcc tgaatctccc tgagggaccc atccagtttg tccccaccat ctccactctg 720
   ccccatgggc tctggcaaat ctctgaggct ggaacagggg tctccagtat attcatctac 780
   catggtgagg tgccccaggc cagccaagta ccccctccca gtggattcac tgtccacggc 840
   ctcccaacat ctccagaccg gccaggctcc accagcccct tcgctccatc agccactgac 900
   ctgcccagca tgcctgaacc tgccctgacc tcccgagcaa acatgacaga gcacaagacg 960
   tcccccaccc aatgcccggc agctggagag gtctccaaca agcttccaaa atggcctgag 1020
   ccggtggagc agttctaccg ctcactgcag gacacgtatg gtgccgagcc cgcaggcccg 1080
   gatggcatcc tagtggaggt ggatctggtg caggccaggc tggagaggag cagcagcaag 1140
   agcctggagc gggaactggc caccccggac tgggcagaac ggcagctggc ccaaggaggc 1200
   ctggctgagg tgctgttggc tgccaaggag caccggcggc cgcgtcgact cgagcgagct 1260
   cccggggggg gttctcatca tcatcatcat cattaataat aaactaatcc ttaacattct 1320
   actcccaacc ccttggggcc tctaaacggg tcttgagggg ttttttg 1367
<210> 56
   <211> 938
   <212> DNA
   <213> Artificial Sequence
<220>
   <223> Description of Artificial Sequence:Expressionskonstrukt
<400> 56
   gaaattaata cgactcacta tagggagacc acaacggttt ccctctagaa ataattttgt 60
   ttaactttaa gaaggagata taccatgaaa tatacatatt ctctgcacgt gatcgtgcag 120
   actagcaaag gagaagaact tttcactgga gttgtcccaa ttcttgttga attagatggt 180
   gatgttaatg ggcacaaatt ttctgtcagt ggagagggtg aaggtgatgc tacatacgga 240
   aagcttaccc ttaaatttat ttgcactact ggaaaactac ctgttccatg gccaacactt 300
   gtcactactt tctcttatgg tgttcaatgc ttttcccgtt atccggatca tatgaaacgg 360
   catgactttt tcaagagtgc catgcccgaa ggttatgtac aggaacgcac tatatctttc 420
   aaagatgacg ggaactacaa gacgcgtgct gaagtcaagt ttgaaggtga tacccttgtt 480
   aatcgtatcg agttaaaagg tattgatttt aaagaagatg gaaacattct cggacacaaa 540
   ctcgagtaca actataactc acacaatgta tacatcacgg cagacaaaca aaagaatgga 600
   atcaaagcta acttcaaaat tcgccacaac attgaagatg gatccgttca actagcagac 660
   cattatcaac aaaatactcc aattggcgat ggccctgtcc ttttaccaga caaccattac 720
   ctgtcgacac aatctgccct ttcgaaagat cccaacgaaa agagagacca catggtcctt 780
   cttgagtttg taacagctgc tgggattaca catggcatgg atgaactata caaacccggg 840
   gggggttctc atcatcatca tcatcattaa taaactaatc cttaacattc tactcccaac 900
   cccttggggc ctctaaacgg gtcttgaggg gttttttg                          938
<210> 57
   <211> 905
   <212> DNA
   <213> Artificial Sequence
<220>
   <223> Description of Artificial Sequence: Expressionskonstrukt
<400> 57
   gaaattaata cgactcacta tagggagacc acaacggttt ccctctagaa ataattttgt 60
   ttaactttaa gaaggagata taccatgact agcaaaggag aagaactttt cactggagtt 120
   gtcccaattc ttgttgaatt agatggtgat gttaatgggc acaaattttc tgtcagtgga 180
   gagggtgaag gtgatgctac atacggaaag cttaccctta aatttatttg cactactgga 240
   aaactacctg ttccatggcc aacacttgtc actactttct cttatggtgt tcaatgcttt 300
   tcccgttatc cggatcatat gaaacggcat gactttttca agagtgccat gcccgaaggt 360
   tatgtacagg aacgcactat atctttcaaa gatgacggga actacaagac gcgtgctgaa 420
   gtcaagtttg aaggtgatac ccttgttaat cgtatcgagt taaaaggtat tgattttaaa 480
   gaagatggaa acattctcgg acacaaactc gagtacaact ataactcaca caatgtatac 540
   atcacggcag acaaacaaaa gaatggaatc aaagctaact tcaaaattcg ccacaacatt 600
   gaagatggat ccgttcaact agcagaccat tatcaacaaa atactccaat tggcgatggc 660
   cctgtccttt taccagacaa ccattacctg tcgacacaat ctgccctttc gaaagatccc 720
   aacgaaaaga gagaccacat ggtccttctt gagtttgtaa cagctgctgg gattacacat 780
   ggcatggatg aactatacaa acccgggggg ggttctcatc atcatcatca tcattaataa 840
   actaatcctt aacattctac tcccaacccc ttggggcctc taaacgggtc ttgaggggtt 900
   ttttg                                                             905

## Description

Die Erfindung betrifft ein Verfahren zur optimierten Herstellung von Proteinen in einem *in vitro* oder *in vivo* Expressionssystem sowie dafür geeignete Reagenzien.

Hannig, G. & Makrides, S.C. (1998) Tibtech Vol 16, pp 54-60, haben Strategien zur Optimierung der heterologen Proteinexpression in E. coli beschrieben. Ein entscheidender Faktor ist hierbei die Effizienz der Initiation der Translation. Die Verwendung bestimmter Codons spielt hierbei eine gewisse Rolle. So konnten George et al. (1985) DNA Vol 4, pp 273-281, zeigen, dass die Expression eines heterologen Genes gesteigert werden kann, wenn man in dem Bereich nach dem Startcodon solche Codons verwendet, die in E. coli Genen häufig benutzt werden. Besonders wichtig für die Translations-Initiation sind hauptsächlich strukturelle Elemente am 5'-Ende der mRNA. Von Makrides (1996) Microbiol. Rev. Vol 60, pp 512-538, wurden verschiedene Translations-Verstärker-Sequenzen, wie beispielsweise eine Sequenz aus dem T7-Phagen Gen 10 Leader und eine U-reiche Sequenz aus der 5 '-untranslatierten Region einiger mRNA 's, wie beispielsweise des E. coli atpE Gens, beschrieben.

Bislang wurden keine universell einsetzbaren Translations-Initiationssequenzen beschrieben. Es wurden jedoch Strategien beschrieben, die das Potential der Sekundärstruktur-Bildung am 5 '-Ende der mRNA reduzieren. Insbesondere wurde die Ribosomen-Bindungsstelle mit Adenin und Thymin Bausteinen angereichert. Stenstöm et al. (2001) Gene Vol 263, pp 273-284, zeigten, dass stark exprimierte E. coli Gene insbesondere bei dem dem Startcodon folgenden + 2 Codon einen hohen Gehalt an Adeninen aufweisen. Allerdings gibt es auch für diese Regel viele positive und negative Ausnahmen.

Schließlich zeigten Pederson-Lane et al. (1997) Protein Expr. Purif. Vol 10, pp 256-262, dass ein hoher GC-Gehalt unmittelbar nach dem Startcodon negativ für die Expression ist und mit Konversion der Purin Basen des 3., 4. und 5. Codons gegen Thymidin Basen die Expression der Thymidylatsynthase auf 25 % des Gesamtproteins gesteigert werden konnte.

Es wird angenommen, dass bei all diesen Maßnahmen die Zugängigkeit der 30S Ribosomenuntereinheit zu der messenger RNA eine entscheidende Rolle spielt. Besonders wichtig ist hierbei der freie Kontakt an die nach Shine und Dalgarno benannte Sequenz unmittelbar vor dem Startcodon und der Kontakt zu dem Startcodon selbst. Sind hingegen diese Sequenzelemente in stabilen RNA-Sekundärstrukturen gebunden, so läuft die Initiation der Translation sehr ineffizient. Tessier et al. (1984) Nucl. Ac. Res. Vol 12, pp 7663-7675, zeigten in einer systematischen Untersuchung, dass durch eine gezielte Mutation diese Form von Stämmchen und Schleifen (sogenannten Stem-Loops oder Hairpin-Loops) ähnelnden Sekundärstrukturen aufgelöst und damit die Effizienz der Translation erheblich gesteigert werden kann. Der Effekt dieser Sekundärstrukturen auf die Translation kann nach deren thermodynamischen Parametern berechnet werden. So bewirkt eine Stabilisierung um 1,4 kcal/mol eine 10fache Reduzierung der Expression (Gold (1988) Ann. Rev. Biochem, Vol 57, pp 199-233) und eine Stabilisierung um 2,3 kcal/mol reduziert die Bindung des Ribosoms um eine Größenordnung (de Smit & van Duin (1994) J. Mol. Biol. Vol 244, pp 144-150).

Von Sprengart et al. (1996) EMBO Vol 15, pp 665-674, wurde als weiterer Translations-Verstärker die sogenannte "Downstream Box", ein Sequenzelement direkt nach dem Startcodon der T7-Gene mit Homologie zur ribosomalen 16S RNA beschrieben. Es wird angenommen, dass dieses Element durch eine Wechsewirkung der beiden homologen Basenpaare die Bindung der 30S Ribosomenuntereinheit verstärkt. Allerdings eignet sich auch dieses Element nicht als universeller Translations-Verstärker.

Die Nachteile bekannter Verfahren liegen darin, dass für jedes neue Gen eine Optimierung der 5'-Region der mRNA entweder in der 5 '-untranslatierten Region oder in der translatierten Region durchgeführt werden muss, um die Codon-Verwendung zu optimieren, oder unerwünschte Sekundärstrukturen der mRNA mit Auswirkung auf die Shine-Dalgarno Sequenz oder das Startcodon zu vermeiden. Dies bedarf im Regelfall einer aufwändigen Analyse der RNA-Struktur mit entsprechenden Programmen (z.B. Mukund et al. (1999) Curr. Science Vol 76, pp 1486-1490, oder Jaeger et al. (1990) Meth. Enzymol. Vol 183, pp 281-306), sowie mehrerer PCR-Amplifikationen und Klonierungsschritte. Will man auf diese Weise eine große Anzahl von Genen, beispielweise aus einer Genbank exprimieren, so ist dazu in jedem Falle eine genaue Kenntnis der Sequenz erforderlich, weshalb diese Methoden nicht auf unbekannte Gene angewendet werden können. Selbst bei Kenntnis der Sequenzen wäre dieses Verfahren wesentlich aufwändiger als eine universell anwendbare Methode.

Ein weiterer Ansatz zur Verstärkung der Translation besteht darin, als universellen Translations-Enhancer ein Fusionsprotein mit einem stark exprimierten Gen zu bilden, an dessen C-terminales Ende das gewünschte Gen gesetzt wird. Als Beispiel für den Erfolg dieser Strategie ist die Fusion mit dem Ubiquitin Gen zu nennen, wie sie von Butt et al. (1989) PNAS Vol 86, pp 2540-2544, durchgeführt wurde.

Auch dieser Ansatz kann jedoch nicht ohne Weiteres auf die Expression beliebiger Gene übertragen werden. Werden nämlich Fusionsproteine verwendet, so wird am N-Terminus des Proteins eine mehr oder weniger große Fusion angefügt, die aufgrund der Größe und Eigenschaften des Fusionspartners mit der Funktion des gewünschten Proteins interferieren können. Je kleiner die Fusionsproteine oder Teile von diesen gewählt werden, desto geringer ist in vielen Fällen auch deren translationssteigernde Wirkung. Bei großen Fusionsproteinen zeigt sich ein weiterer Nachteil prokaryontischer Expressionssysteme: Es steigt gleichzeitig die Wahrscheinlichkeit für unvollständige Transkription oder Translation durch vorzeitige Termination oder interne Initialisierung. Auch die Wahrscheinlichkeit für proteolytischen Abbau ist erhöht.

Es besteht somit ein Bedürfnis, ein Verfahren zur optimierten Herstellung von Proteinen bereitzustellen, bei dem die Nachteile des Standes der Technik zumindest teilweise beseitigt sind.

Ein Gegenstand der Erfindung ist ein Verfahren zur Herstellung eines Proteins, umfassend die Schritte:
(a) Bereitstellen einer für das Protein kodierenden Nukleinsäuresequenz, wobei 3'-seitig des Translations-Startcodons eine heterologe Nukleinsäuresequenz im korrekten Leseraster eingefügt wird, die so gewählt wird, dass in einem Abstand von 6-30 Nukleotiden 3'-seitig des Translations-Startcodons eine Stem-Loop-Struktur ausgebildet wird,
(b) Bereitstellen eines zur Expression des Proteins geeigneten Expressionssystems und
(c) Einbringen der Nukleinsäuresequenz gemäß (a) in das Expressionssystem gemäß (b) unter Bedingungen, dass das Protein synthetisiert wird.

Die erfindungsgemäße Lösung für ein universell optimiertes Expressionskonstrukt besteht darin, dass ein kleines heterologes DNA-Sequenzelement mit vorzugsweise maximal 201 Basenpaaren, besonders bevorzugt maximal 45 Basenpaaren, unmittelbar nach dem Startcodon des zu exprimierenden Gens eingefügt wird, welches die Ausbildung stabiler Stem-Loop-Strukturen im Bereich der Shine-Dalgarno-Sequenz und des Startcodons weitgehend verhindert und dadurch zur optimierten Translations-Initiation und optimierter Proteinsynthese führt. Es wird somit ein Fusionsprotein gebildet, wobei vorzugsweise nur ein kleines Peptid mit maximal 67 Aminosäuren und besonders bevorzugt maximal 15 Aminosäuren an das gewünschte Protein angefügt wird.

Eine wichtige Vorraussetzung für das heterologe DNA-Sequenzelement ist, dass es im korrekten Leseraster eingefügt wird, d.h. dass keine Rasterverschiebung im zu exprimierenden Gen stattfindet. Eine weitere wichtige Eigenschaft des heterologen DNA-Sequenzelements ist, dass sich in der transkribierten RNA in einem Abstand von 6-30 Basen, vorzugsweise 12-21 Basen, hinter dem Startcodon eine stabile Stem-Loop-Struktur ausbilden kann, wobei die Basenpaarung in der Stem-Loop-Struktur zumindest teilweise durch die eingefügte Sequenz bewirkt wird. Diese Stem-Loop-Struktur soll so beschaffen sein, dass sie nach der erfolgten Initiation der Translation durch das Ribosom wieder aufgelöst werden kann und somit nicht zu einem Abbruch der Translation führt. Diese durch Einführung der heterologen Nukleinsäuresequenz in das Expressionskonstrukt entstandete Stem-Loop-Struktur kann sich bei nahezu jedem Gen in der gleichen Weise ausbilden und dadurch verhindern, dass die für die Translations-Initiation wichtigen Sequenzen vor dem Loop größere Sekundärstrukturen mit der kodierenden Sequenz des Gens eingehen können. Der Bereich unmittelbar vor dieser Stem-Loop-Struktur und nach dem Startcodon ist vorzugsweise eine Sequenz ohne Sekundärstruktur und kann auch keine Sekundärstruktur mit der 5'-untranslatierten Region ausbilden. Besonders bevorzugt ist in dieser Region eine GC-arme Sequenz, da bei einer derartigen Sequenz die Ausbildung stabiler Sekundärstrukturen mit Sequenzen innerhalb der translatierten Region minimiert wird.

Das heterologe Nukleinsäure-Sequenzelement kann in die Zielsequenz, z.B. in einen Plasmidvektor zur Expression von heterologen Genen mit bekannten Klonierungs- oder/und Amplifikationstechniken eingefügt werden. Möglich ist z.B. der Aufbau dieser Sequenz durch PCR-Primer zur Klonierung des gewünschten Gens oder durch Primer, mit denen DNA-Expressionskonstrukte für die *in vitro* Proteinexpression hergestellt werden.

Das erfindungsgemäße Verfahren kann zur Herstellung und gegebenenfalls Gewinnung von Proteinen in *in vitro* Expressionssystemen eingesetzt werden. Beispiele für geeignete *in vitro* Expressionssysteme sind prokaryontische *in vitro* Expressionssysteme, wie etwa Lysate von gram-negativen Bakterien, beispielsweise von *Escherichia coli,* oder gram-positiven Bakterien, wie beispielsweise *Bacillus subtilis,* oder eukaryontische *in vitro* Expressionssysteme, wie etwa Lysate von Säugerzellen, wie beispielsweise von Kaninchen, Reticulocyten, humanen Tumorzelllinien, Hamsterzelllinien, oder anderen Wirbeltierzellen, wie beispielsweise Oozyten und Eiern von Fischen und Amphibien, sowie Insektenzelllinien, Hefezellen, Algenzellen oder Extrakte aus Pflanzenkeimen.

Alternativ kann die Herstellung des Proteins in einem *in vivo* Expressionssystem erfolgen, wobei eine prokaryontische Zelle, z.B. eine gram-negative prokaryontische Wirtszelle, insbesondere eine E. coli Zelle, oder eine gram-positive prokaryontische Zelle, insbesondere eine *Bacillus subtilis* Zelle, eine eukaryontische Wirtszelle, z.B. eine Hefezelle, eine Insektenzelle oder eine Wirbeltierzelle, insbesondere eine Amphibien-, Fisch-, Vogel- oder Säugerzelle, oder ein nicht-humaner eukaryontischer Wirtsorganismus als Expressionssystem verwendet werden kann.

Die Einführung der heterologen Nukleinsäure-Sequenz in die für das gewünschte Protein kodierenden Nukleinsäure-Sequenz kann durch Standardmethoden der Molekularbiologie, z.B. durch Klonierung, wie etwa Restriktionsspaltung oder/und Ligation, durch Rekombination oder/und durch Nukleinsäureamplifikation erfolgen. Die Nukleinsäure-Zielsequenz kann dabei auf einem geeigneten Vektor, z.B. einem Plasmidvektor zur Expression von heterologen Genen, oder auf einem Konstrukt für eine *in vitro* Proteinexpression vorliegen. Besonders bevorzugt ist eine Nukleinsäureamplifikation in einem oder mehreren Schritten, wobei durch Auswahl geeigneter Primer die heterologe Nukleinsäuresequenz und gegebenenfalls Expressionskontrollsequenzen, wie etwa Promotor, ribosomale Bindungensstellen und Terminatoren, an die für das gewünschte Protein kodierende Nukleinsäure-Sequenz angefügt werden. Besonders bevorzugt ist eine Zweistufen-PCR, wobei in einer ersten Stufe zumindest ein Teil der heterologen Nukleinsäure-Sequenz an eine Nukleinsäure-Zielsequenz, die für das gewünschte Protein kodiert, und in einem zweiten Schritt Expressionskontrollsequenzen angefügt werden. Eine bevorzugte Ausführungsform zur Durchführung einer Zweischritt-PCR ist in den Beispielen exemplarisch dargestellt.

Die heterologe Nukleinsäure-Sequenz, die in der Lage ist, eine Stem-Loop-Struktur 3'-seitig des Translations-Startcodons auszubilden, wird im korrekten Leseraster 3'-seitig des Translations-Startcodons, üblicherweise des ersten ATG-Codons, in die für das gewünschte Protein kodierende Nukleinsäure-Sequenz eingefügt. Vorzugsweise erfolgt die Einfügung in einem Abstand bis zu 6 Nukleotiden, besonders bevorzugt unmittelbar nach dem Translations-Startcodon. Eine Einfügung im "korrekten Leseraster" bedeutet dabei, dass keine Verschiebung des Leserahmen in der Proteinkodierenden Nukleinsäure-Sequenz erfolgt. Dies bedeutet wiederum, dass die Länge der heterologen Nukleinsäure-Sequenz in Nukleotiden ein Vielfaches von 3 ist. Bevorzugt ist die Länge im Bereich von 6-201 Nukleotiden, besonders bevorzugt im Bereich von 12-45 Nukleotiden.

Die Einfügung der heterologen Nukleinsäure-Sequenz in die Protein-kodierende Nukleinsäure-Sequenz erfolgt derart, dass eine Stem-Loop-Struktur in einem geeigneten Abstand 3'-seitig des Translations-Codons ausgebildet wird. Der Abstand (zwischen dem letzten Nukleotid des Translationsstart-Codons und dem ersten Nukleotid des Stem) beträgt günstigerweise 6-30 Nukleotide, besonders bevorzugt 12-21 Nukleotide. 5'-seitig derjenigen Sequenzen, die für die Bildung der Stem-Loop-Struktur vorgesehen sind, enthält die heterologe Nukleinsäure-Sequenz vorzugsweise einen AT-reichen Bereich, d.h. einen Bereich mit einem AT-Gehalt > 50 %, insbesondere > 60 %.

Die Länge des Stems in der Stem-Loop-Struktur liegt vorzugsweise im Bereich von 4 bis 12 Nukleotiden, besonders bevorzugt von 5 bis 10 Nukleotiden. Der Stem der Stem-Loop-Struktur enthält vorzugsweise zwei vollständig zueinander komplementäre Abschnitte. Es können jedoch auch ein oder mehrere Basenfehlpaarungen vorhanden sein, sofern dadurch die Stabilität nicht zu stark verringert wird. Die Basenpaarungen im Stem können AT- und GC-Basenpaarungen und Kombinationen davon sein. Ein Anteil der GC-Basenpaarungen von > 50 % ist bevorzugt. Die Länge des Loops beträgt vorzugsweise 2 bis 8 Nukleotide, sie ist jedoch nicht sonderlich kritisch. Die thermodynamische Stabilität der Stem-Loop-Struktur ist zweckmäßigerweise hoch genug, dass die Ausbildung einer Sekundärstruktur im Bereich des ATG-Startcodons, der 15 5'-liegenden Nukleotide, welche die Shine-Dalgarno-Sequenz beinhalten, und mindestens der 5 3'-befindlichen Nukleotide verhindern. Andererseits sollte die thermodynamische Stabilität der Stem-Loop-Struktur nicht so hoch sein, dass die Prozessierung des Ribosoms auf der mRNA behindert wird. Vorzugsweise liegt die thermodynamische Stabilität der Stem-Loop-Struktur im Bereich von -4 bis -15 kcal/mol.

Die zur Expression des gewünschten Proteins verwendeten Expressionskontrollsequenzen umfassen Promotoren, ribosomale Bindungsstellen, d.h. Shine-Dalgarno-Sequenzen für prokaryontische Expressionssysteme bzw. Kozak-Sequenzen für eukaryontische Expressionssysteme, Enhancer, Terminatoren, Polyadenylierungs-Sequenzen etc. Dem Fachmann sind entsprechende Expressionskontrollsequenzen aus Standardlehrbüchern der Molekularbiologie, z.B. Sambrook et al. (1989) Molecular Cloning, A Laboratory Manual, Cold Spring Harbor oder Ausubel et al. (1989) Current Protocols in Molecular Biology, John Wiley & Sons, New York, bekannt.

Weiterhin kann die heterologe Nukleinsäuresequenz auch Abschnitte enthalten, die für eine Aufreinigungsdomäne, z.B. eine Poly-His-Domäne, eine FLAG-Epitop-Domäne etc., oder/und für eine Proteinase-Erkennungsdomäne, z.B. eine IgA-Protease- oder Faktor-X-Domäne, kodieren. Durch die Aufreinigungsdomäne kann die Gewinnung des gewünschten Proteins, z.B. aus einem *in vitro* Translationsansatz oder einer Wirtszelle bzw. dem zur Kultivierung verwendeten Medium, vereinfacht werden. Durch Protease-Spaltung innerhalb der Protease-Erkennungsdomäne kann die heterologe Peptidsequenz von dem gewünschten Protein abgespalten werden.

Die heterologe Nukleinsäuresequenz oder/und die für das gewünschte Protein kodierende Nukleinsäuresequenz werden - um eine weitere Verbesserung der Expressionshöhe zu erreichen - günstigerweise so gewählt, dass sie zumindest teilweise eine an das jeweilige Expressionssystem angepasste Codon-Nutzung aufweisen.

Ein weiterer Gegenstand der Erfindung ist ein Reagenz zur Herstellung eines Proteins, umfassend
(a) eine zu der für das gewünschte Protein kodierenden Nukleinsäuresequenz heterologe Nukleinsäuresequenz, die im korrekten Leseraster in die Protein-kodierende Nukleinsäuresequenz eingefügt werden kann, und die in einem Abstand von 6-30 Nukleotiden 3'-seitig des Transtations-Startcodons eine Stem-Loop-Struktur ausbilden kann, und
(b) ein zur Herstellung des Proteins geeignetes Expressionssystem.

Die heterologe Nukleinsäuresequenz kann in Form einer vollständigen Sequenz oder in Form von mehreren Teilsequenzen vorliegen.

Das erfindungsgemäße Verfahren und Reagenz sind insbesondere für die Synthese von Proteinen schwer exprimierbarer Gene sowie der Synthese von Proteinen ausgehend von Genbanken anwendbar, da hierbei die Erfolgsrate gegenüber der bei der Verwendung üblicher Expressionsvektoren gesteigert werden kann.

Weiterhin soll die vorliegende Erfindung durch die nachfolgenden Abbildungen und Beispiele näher erläutert werden.

Es zeigen:
**Figur 1** eine schematische Darstellung der zur Durchführung einer Zweischritt-PCR notwendigen Nukleinsäure-Sequenzelemente;
**Figur 2** eine schematische Darstellung von unterschiedlich langen Stem-Loop-Strukturen in zur Einfügung in GFP Expressionkonstrukte verwendeten heterologen Nukleinsäure-Sequenzen;
**Figur 3** eine Auswertung der Ergebnisse bei der Expression von GFP unter Verwendung der Hairpin-Loop-GFP-Konstrukte gemäß Figur 3 in einem RTS-Expressionssystem. 1 *µ*l jedes Ansatzes (Doppelbestimmungen) wurde elektrophoretisch über SDS-PAGE aufgetrennt und auf eine PVDF-Membran geblottet. Die Detektion erfolgte über DCP-Star und Lumi-Imager;
**Figur 4** eine schematische Darstellung von unterschiedlichen positionierten Stem-Loop-Strukturen in zur Einfügung von GFP-Expressionskonstrukte verwendeten heterologen Nukleinsäuresequenzen;
**Figur 5** die Expression von GFP unter Verwendung der in Figur 4 dargestellten heterologen Nukleinsäure-Sequenzen. Die Durchführung und Auswertung der Ansätze erfolgte wie in der Legende zur Figur 3 beschrieben;
**Figur 6** eine Auswertung der Ergebnisse bei der Expression des CIITA Gens (Wildtyp: Spur 1; Mutanten Spuren 2-10) unter Verwendung unterschiedlicher heterologer Nukleinsäuresequenzen mit Stem-Loop-Strukturen;
**Figur 7** eine Auswertung der Ergebnisse bei der Expression des CMV Capsid (1049) Gens (Wildtyp: Spur 1; Mutanten Spuren 2-10) unter Verwendung unterschiedlicher heterologer Nukleinsäuresequenzen mit Stem-Loop-Strukturen;
**Figur 8** eine Auswertung der Ergebnisse bei der Expression des Survivin Gens (Wildtyp: Spur 10; Mutanten Spuren 1-9) unter Verwendung unterschiedlicher heterologer Nukleinsäuresequenzen mit Stem-Loop-Strukturen;
**Figur 9** eine Auswertung der Ergebnisse bei der Expression des GFP Gens (Wildtyp: Spur 10; Mutanten Spuren 1-9) unter Verwendung unterschiedlicher heterologer Nukleinsäuresequenzen mit Stem-Loop-Strukturen;

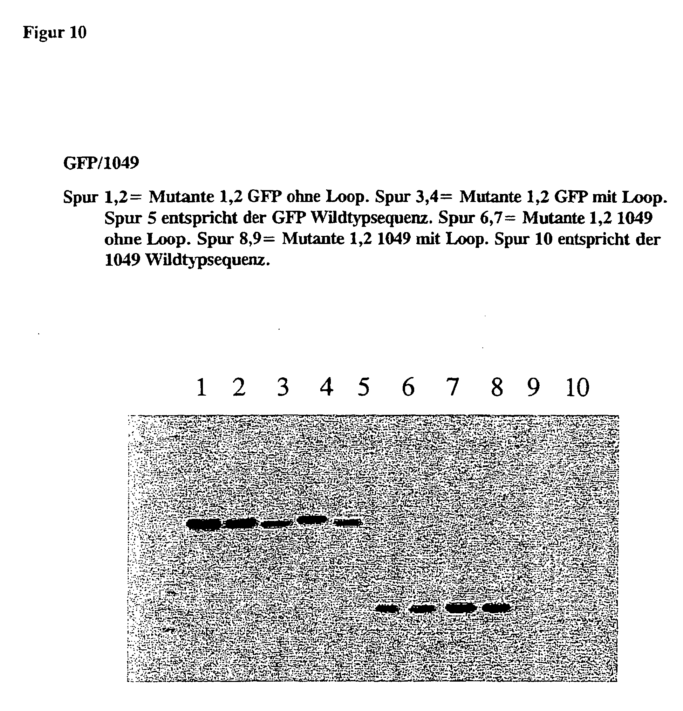

**Figur 10** eine Auswertung der Ergebnisse bei der Expression des GFP und des 1049 Gens unter Verwendung unterschiedlicher heterologer Nukleinsäuresequenzen mit und ohne Stem-Loop-Strukturen;

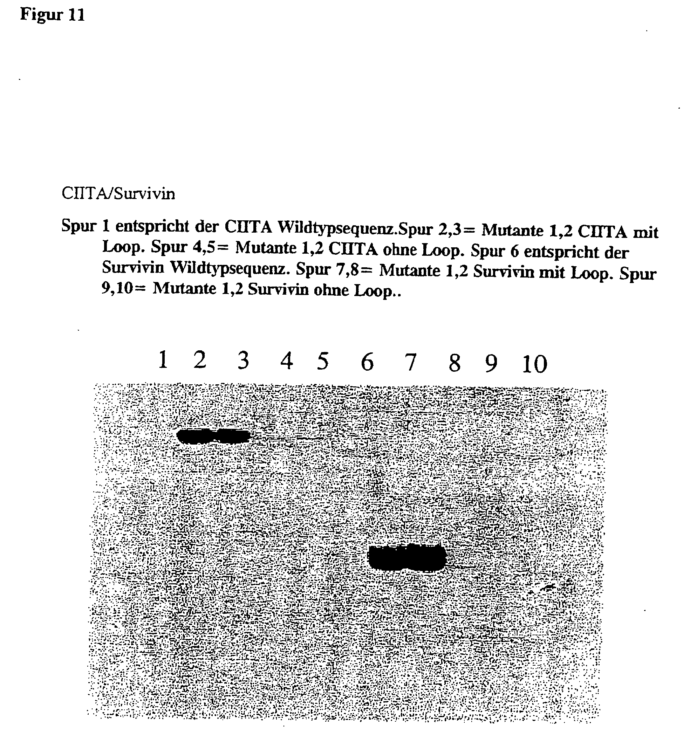

**Figur 11** eine Auswertung der Ergebnisse bei der Expression des CIITA und des Survivin Gens unter Verwendung unterschiedlicher heterologer Nukleinsäuresequenzen mit und ohne Stem-Loop-Strukturen;
**Figur 12** eine schematische Darstellung von zwei unterschiedlichen Stem-Loop-Strukturen in den erfindungsgemäßen heterologen Sequenzen;
**Figur 13** eine Auswertung der Ergebnisse mit den in Figur 12 gezeigten Stem-Loop-Strukturen;
**Figur 14** eine Darstellung der *in vivo* Protein Expression von RNA-Stem-Loop-Kontrukten im Vergleich zu den Wildtyp-Genen im Western Blot. Expression von drei unabhängigen Klonen der RNA-Stem-Loop-Mutanten des CMV Kapsid Proteins 1049 (Spur 1 bis 3) und des CMV Kapsid Proteins 1049 Wildtyp (Spur 4 bis 6). Expression von unabhängigen Klonen von Survivin RNA-Stem-Loop-Mutanten (Spur 7 bis 9) und des Survivin Wildtyps (Spur 10,11)

### Beispiele

### Beispiel 1: Zweischritt-PCR

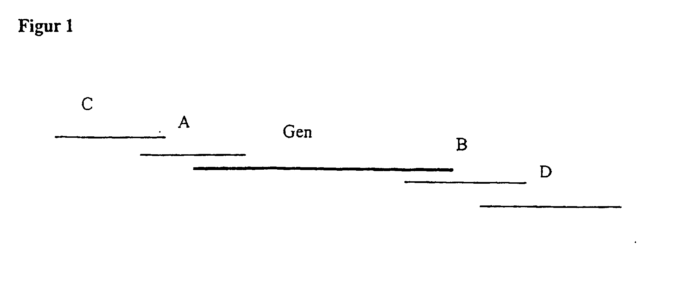

Mit Hilfe einer Zweischritt-PCR können zu exprimierende Gene amplifiziert und mit den entsprechenden Kontrollregionen, wie T7-Promotor, T7 Gen10-Leader (g10), Ribosomaler Bindungsstelle (RBS) und T7-Terminator, versehen werden. Im ersten Schritt wird mittels eines Paares von Primern (A, B), die jeweils auf 15 Basen Länge mit dem entsprechenden Gen komplementär sind und 15 weitere Basen enthalten, die komplementär zu einem zweiten Primerpaar (C, D) sind, das Gen amplifiziert. Das zweite Primerpaar enthält alle wichtigen regulatorischen Elemente, die somit bei einer zweiten PCR-Amplifikation an das Gen angehängt werden (siehe Figur 1).

Über den A-Primer können bei dieser Methode Veränderungen im 5 -Bereich des Gens eingeführt werden. Bei den Hairpin-Loop-Konstrukten wurden über diesen A-Primer Hairpin-Loops mit unterschiedlicher Länge des Hairpin-Loop-Stamms und in unterschiedlicher Position hinter dem Start-Codon in die Gen-Sequenz inseriert.

### Primer C (SEQ ID NO. 1)

### Primer D (SEQ ID NO. 2)

### Reaktionsbedingungen

Die PCR-Reaktionen wurden üblicherweise nach folgendem Schema mit dem Expand High Fidelity Kit (Roche Applied Science) im 50 *µ*l Maßstab durchgeführt:
- PCR 1:: Template 10ng/Ansatz; Primer A 20 pmol/Ansatz; Primer B 20 pmol/Ansatz 95 ° C 5 min + 20 mal (95 °C 1 min + 55 1 min + 72 ° C 1 min) + 4 ° C
- PCR 2:: 2 *µ*l PCR 1; Primer C 20 pmol/Ansatz; Primer D 20 pmol/Ansatz 95 ° C 5 min + 30 mal (95 °C 1 min + 50 ° C 1 min + 72 °C 1 min) + 72 °C 10 min +4 °C

### Beide PCR-Reaktionen wurden jeweils durch Agarose-Gel-Elektrophorese überprüft und die PCR-Produkte der PCR 2 gleichzeitig mit Hilfe eines DNA-Längenstandards, welcher definierte DNA-Mengen enthält, im Lumi-Imager-System quantifiziert. Die daraus erhaltenen PCR-Produkte wurden direkt als Template in RTS-Expressions-Ansätzen eingesetzt.

### Beispiel 2: Expression mit dem RTS in vitro Expressionssystem

Die Expressionen mit dem RTS 100 HY Kit (Firma- Roche Applied Science) wurden in Batch-Ansätzen zu je 50 *µ*l nach der dem Kit beiliegenden Anleitung durchgeführt. Dabei wurden DNA-Mengen von 0,25-1 *µ*g pro Reaktions-Ansatz eingesetzt. Um die Ergebnisse einer Versuchsreihe vergleichen zu können, wurden immer gleiche Mengen des jeweiligen Templates eingesetzt. Die Ansätze wurden bei 30 °C für 4 h inkubiert.

### Beispiel 3: Expression von Hairpin-Loop-GFP-Konstrukten

Es wurde am Beispiel von GFP (Grün-Fluoreszenz-Protein) untersucht, welchen Einfluss Hairpin-Loops (Haarnadel-förmige Schleifen) in der mRNA direkt nach dem Start-ATG ausüben. Dazu wurden RNA-Sequenzen ermittelt, die Hairpin-Loops (HL) mit unterschiedlichen Stammlängen ausbilden. Je länger der Stamm des Hairpin-Loops ist, um so energetisch stabiler ist diese Struktur. Bei der Erstellung der Hairpin-Loops wurde darauf geachtet, dass nur in *E. coli* Genen häufig zu findende Codons verwendet wurden. Die ermittelten Sequenzen für die verschiedenen Hairpin-Loops wurden mittels mRNA-Sekundärstrukturanalyse auf ihre Stabilität im gesamten Konstrukt hin überprüft. Für Sequenzen, die genügend Stabilität aufwiesen, wurden Primer erstellt, die in der beschriebenen Zweischritt-PCR gemäß Beispiel 1 eingesetzt wurden.

### Primer A:

### ohne Hairpin-Loop (SEQ ID NO. 3)

### Stammlänge 4 bp (SEQ ID NO.4)

### Stammlänge 5 bp (SEQ ID NO. 5)

### Stammlänge 6 bp (SEQ ID NO. 6)

### Stammlänge 7 bp (SEQ ID NO. 7)

### Stammlänge 8 bp (SEQ ID NO. 8)

### Primer B (SEQ ID NO. 9)

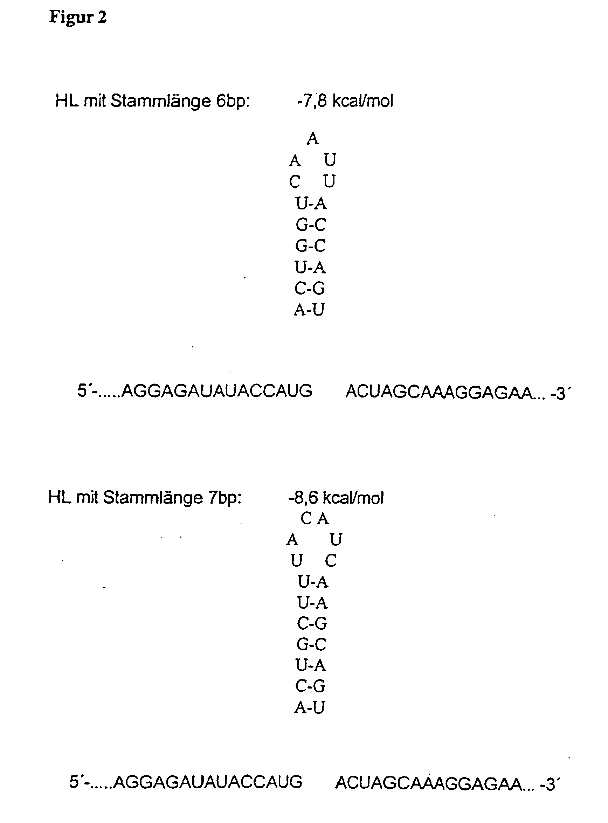

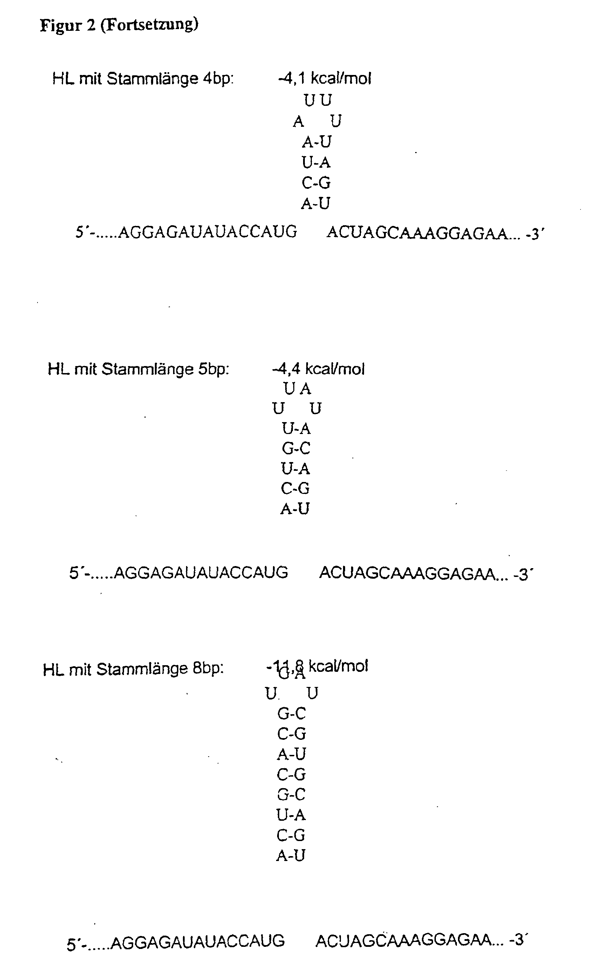

Eine schematische Darstellung der mRNA-Sekundärstrukturen der Hairpin-Loop-GFP-Konstrukte ist in Figur 2 gezeigt.

### RTS Expression

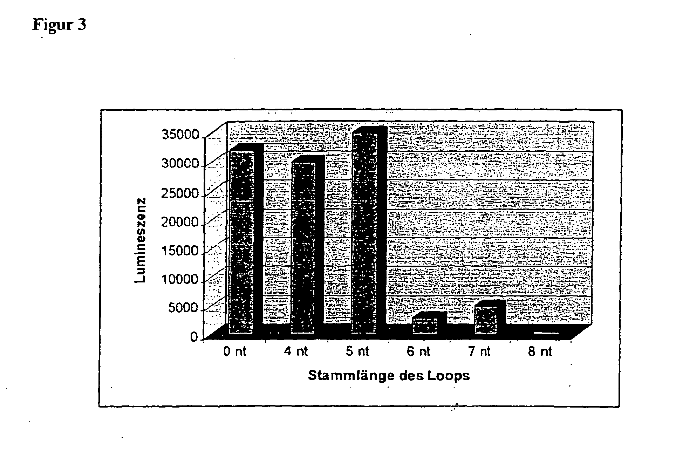

Nach Expression im RTS gemäß Beispiel 2 wurde zur Verifizierung die gebildete GFP-Menge im Fluorimeter gemessen und der Western-Blot mittels CDP-Star-Detektion und Auswertung im Lumi-Imager quantitativ analysiert. Die Ergebnisse sind in Figur 3 gezeigt.

Es ist eindeutig erkennbar, dass die Expressionsrate mit der Stammlänge des Hairpin-Loops variiert. Bis zu einer Stammlänge von 5 bp ist die Expressionsrate relativ konstant, um dann anschließend abzufallen. Bei einer Stammlänge von 8 bp ist nahezu keine Expression mehr nachweisbar. Diese Untersuchungen bestätigen die oben erhaltenen Ergebnisse. Man kann also sagen, dass ein Hairpin-Loop mit einer Stammlänge ab 6 bp oder besser mit einer Freien Energie von -7,8 kcal/mol eine Struktur darstellt, die einen erheblichen Einfluss auf die Expression ausübt. Zu erklären ist dies damit, dass diese Struktur bei den Expressionsbedingungen stabil ist und somit das vorgelagerte Start-ATG nicht frei zugänglich ist.

### Beispiel 4: Ermittlung des minimalen Abstandes zum Start-ATG

Um nun zu bestimmen, bis zu welchem Abstand ein solcher Hairpin-Loop einen Einfluss auf die Expression ausübt, wurde der Hairpin-Loop mit der Stammlänge 8 bp (Energie -11,8 kcal/mol) in Schritten zu je 3 Basen vom Start-ATG weg in die GFP-Sequenz verschoben. Die Sequenzen der auf diese Weise erhaltenen A-Primer war wie folgt:

### Stammlänge 8 bp, 6 Basen in die GFP-Sequenz hineinverschoben (SEQ ID NO. 10):

### Stammlänge 8 bp, 9 Basen in die GFP-Sequenz hineinverschoben (SEQ ID NO. 11 ):

### Stammlänge 8 bp, 12 Basen in die GFP-Sequenz hineinverschoben (SEQ ID NO.12):

### Stammlänge 8 bp, 15 Basen in die GFP-Sequenz hineinverschoben (SEQ ID NO. 13):

### Stammlänge 8 bp, 18 Basen in die GFP-Sequenz hineinverschoben (SEQ ID NO. 14):

### Stammlänge 8 bp, 21 Basen in die GFP-Sequenz hineinverschoben (SEQ ID NO. 15):

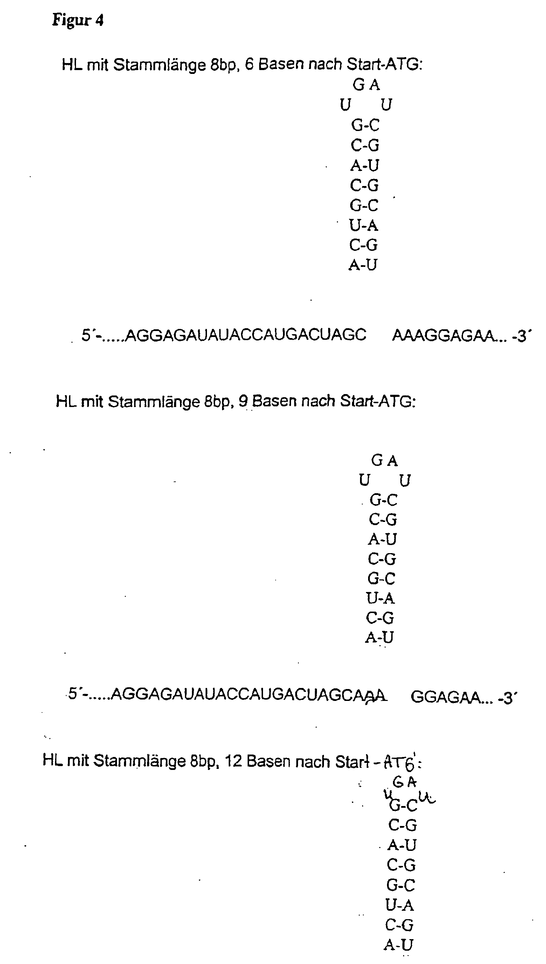

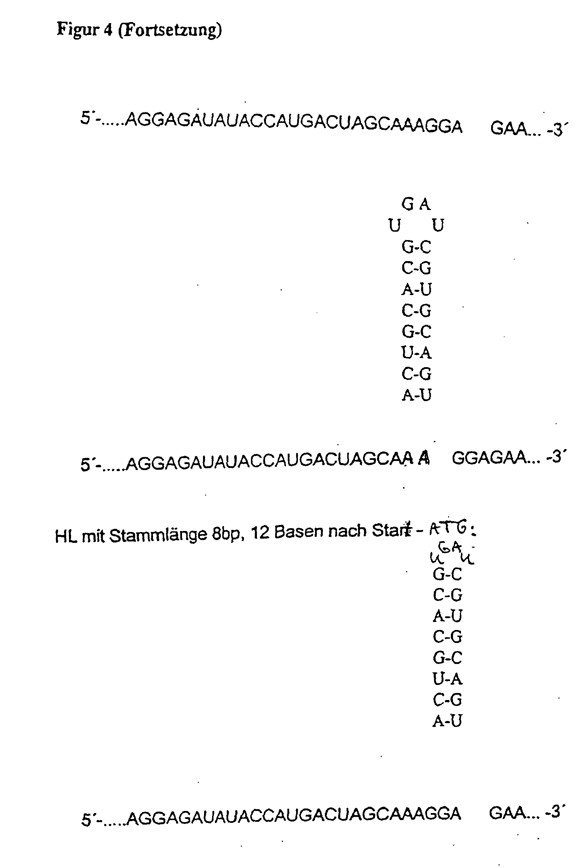

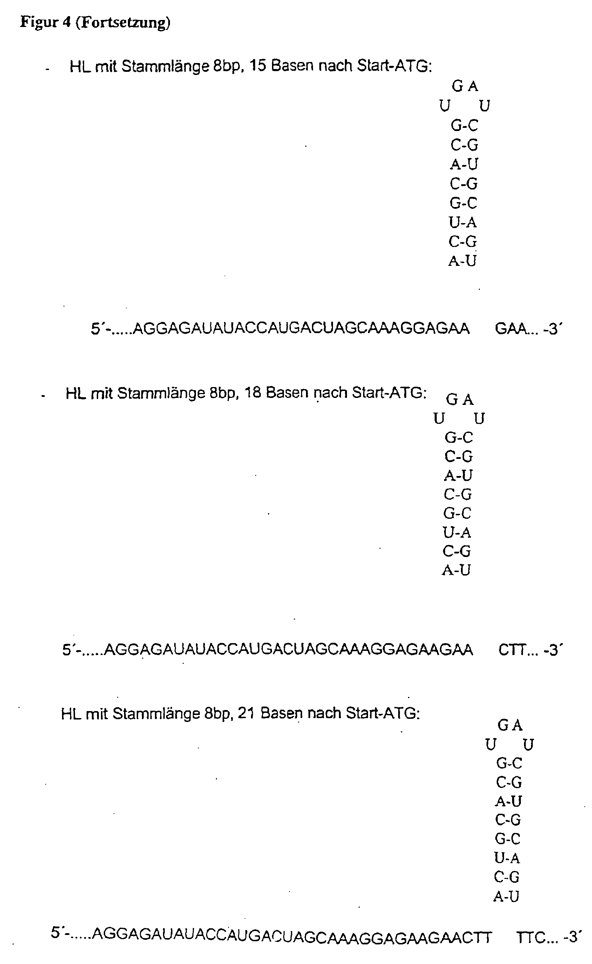

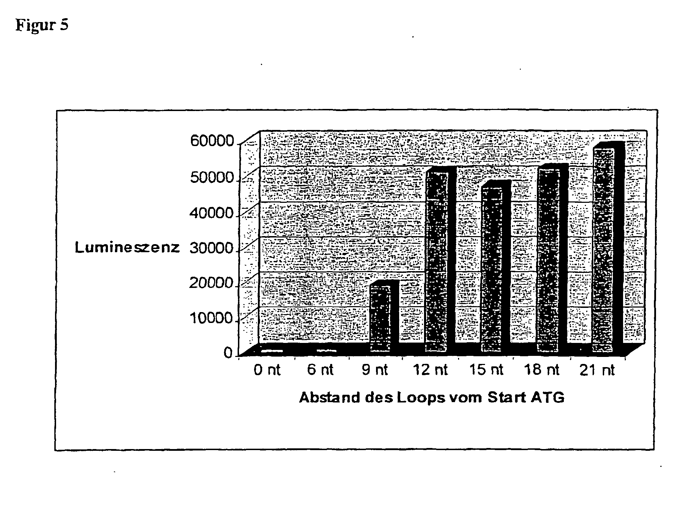

Diese DNA-Konstrukte mit den in Figur 4 gezeigten Sekundärstrukturen wurden ebenfalls über Zweischritt-PCR mit den zuvor beschriebenen Primern B, C und D synthetisiert und direkt aus der PCR-Reaktion in Expressionsansätzen als Template eingesetzt. Dabei wurde durch Quantifizierung über ein Agarose-Gel mit DNA-Marker VII und Auswertung dieses Gels im Lumi-Imager sichergestellt, dass gleiche Mengen an Template eingesetzt wurden. Die Expressionsansätze wurden über einen Western-Blot ausgewertet. Die Ergebnisse sind in Figur 5 gezeigt.

Die Expressionen lassen erkennen, dass ab einem Abstand von 9 Basen zum Start-ATG die Translation der mRNA möglich ist. Es besteht aber noch ein hemmender Einfluss des Hairpin-Loops. Erst ab einem Abstand von 12 Basen verläuft die Translation wieder nahezu ungehemmt. Man kann also aus diesen Ergebnissen schließen, dass das Ribosom einen Platzbedarf von 9-11 Basen nach dem Start-ATG besitzt. Des Weiteren lässt sich aus diesen Ergebnissen folgern, dass ein Hairpin-Loop, der 12 oder mehr Basen nach dem Start-ATG entfernt ist, zwar einen Einfluss auf die mRNA-Sekundärstruktur aber keinen auf die Initiation der Expression hat.

### Beispiel 5: Einführung von Stem-Loop-Strukturen zur Auflösung ungünstiger Sekundärstrukturen

Bei früheren Expressionsansätzen mit dem Rapid Translation System (Roche Applied Science) wurde bei einigen Genen nur eine geringe oder gar keine Expression gefunden. Als Ursache hierfür wurde oft eine ungünstige RNA-Sekundärstruktur ermittelt, bei der entweder das Startcodon, oder die Shine-Dalgarno-Sequenz in einer Sekundärstruktur mit der Gensequenz involviert war, und damit in gebundener Form vorlag.

Für drei dieser Gene, Survivin, Cytomegalovirus Capsid Protein 1049 (1049) und Class II Transactivator (CIITA) wurde eine heterologe Nukleinsäuresequenz mit einem Hairpin-Loop und einer Stammlänge von 7 Basen in einem Abstand von 1 5 Basen nach dem Startcodon eingeführt. Direkt anschließend an den Hairpin Loop wurde das Wildtyp-Gen (siehe unten *) ohne das Start ATG gesetzt. Vor den Hairpin- Loop wurden jeweils AT reiche Sequenzen gesetzt, welche weniger stabile Basenpaarungen als GC reiche Sequenzen ausbilden können. Außerdem wurde darauf geachtet keine für E. coli seltenen Codons innerhalb der eingeführten Sequenzen zu verwenden.

Dadurch, dass nun einerseits ein stabiler idealer Hairpin-Loop vorgegeben wird und andererseits direkt nach dem Startcodon eine Sequenz folgt, welche nicht zu Sekundärstrukturbildung neigt, sollte unabhängig vom nachfolgenden Gen eine freie Zugängigkeit für den Initiationskomplex mit der kleinen ribosomale Untereinheit an die Shine-Dalgarno-Sequenz und das Start-ATG ermöglicht werden.

Es wurden jeweils 9 verschiedene AT-reiche Sequenzen vor den Hairpin-Loops eingesetzt und mit den Wildtyp-Genen* verglichen. Als Kontrollgen wurde das GFP cycle 3 Protein mit den gleichen Hairpin-Loops und AT-reichen Sequenzen über die in Beispiel 1 erwähnte Zweischritt-PCR synthetisiert. Die Sequenzen der A und B Primer sind unten angegeben. In Primer 1 wurden jeweils die homologen Bereiche zu Primer C unterstrichen. Die AT-reiche Sequenz wurde in kursiv, der Hairpin-Loop in Fettdruck und die Wildtyp Gensequenz in fett und unterstrichen angegeben. In Primer B wurden die zu Primer D homologen Bereiche unterstrichen und die Bereiche mit Homologie zum Wildtyp Gen fett angegeben. Als Primer D wurde abweichend zu Beispiel 1 der folgende Primer benutzt:

### Primer D (SEQ ID NO 16):

Die unterstrichene Region ist homolog zu Primer C.

### Varianten Primer A:

### 1049 - 1 (SEQ ID NO. 17):

### 1049 - 2 (SEQ ID NO: 18):

### 1049 - 3 (SEQ ID NO. 19):

### 1049 - 4 (SEQ ID NO. 20):

### 1049 - 5 (SEQ ID NO, 21):

### 1049 - 6 (SEQ ID NO. 22):

### 1049 - 7 (SEQ ID NO. 23):

### 1049 - 8 (SEQ ID NO. 24):

### 1049 - 9 (SEQ ID NO. 25):

### 1049 - 10 (Wildtyp) (SEQ ID NO. 26):

### 1049 - Primer B (SEQ ID NO. 27):

Unterstrichenes ist homolog zu Primer D

### Varianten Primer A:

### Survivin - 1 (SEQ ID NO. 28):

### Survivin - 2 (SEQ ID NO. 29):

### Survivin - 3 (SEQ ID NO. 30):

### Survivin - 4 (SEQ ID NO. 31):

### Survivin - 5 (SEQ ID NO. 32):

### Survivin - 6 (SEQ ID NO. 33):

### Survivin - 7 (SEQ ID NO. 34):

### Survivin - 8 (SEQ ID NO. 35):

### Survivin - 9 (SEQ ID NO. 36):

### Survivin - 10 (A Wildtyp) (SEQ ID NO. 37):

### Survivin - Primer B (SEQ ID NO. 38):

### CIITA - 1 (SEQ ID NO: 39):

### CIITA - 2 (SEQ ID NO. 40):

### CIITA - 3 (SEQ ID NO. 41 ):

### CIITA - 4 (SEQ ID NO: 42:

### CIITA - 7 (SEQ ID NO. 45):

### CIITA - 8 (SEQ ID NO. 46):

### CIITA - 9 SEQ ID NO. 47):

### CIITA - 10 (A Wildtyp) (SEQ ID NO: 48):

### CIITA Primer B (SEQ ID NO. 49):

Im Folgenden sind die Sequenzen der über PCR erzeugten Expressionskonstrukte für die Mutante 1 und den Wildtyp angegeben. Die Wildtyp-Gensequenz ist fett angegeben. Am Ende des Gens wurde mit dem B-Primer jeweils ein hexa-Histidin-Tag zur Detektion mit einem spezifischen Antikörper eingeführt (unterstrichen).

### 1049 - 1 (431 bp) (SEQ ID NO. 50):

### 1049 - 10 (WildTyp) (398 bp) (SEQ ID NO. 51):

### Survivin - 1 (632 bp) (SEQ ID NO. 52):

### Survivin - 10 (Wildtyp) (599 bp) (SEQ ID NO. 53):

### CIITA - 1 (1400 bp) (SEQ ID NO. 54):

### CIITA -10 WT 1367 bp (SEQ ID NO. 55):

### GFP CYC3 - 1 (938 bp) (SEQ ID NO. 56):

### pIVEX-GFP CyC3 - 10 905 bp (SEQ ID NO. 57):

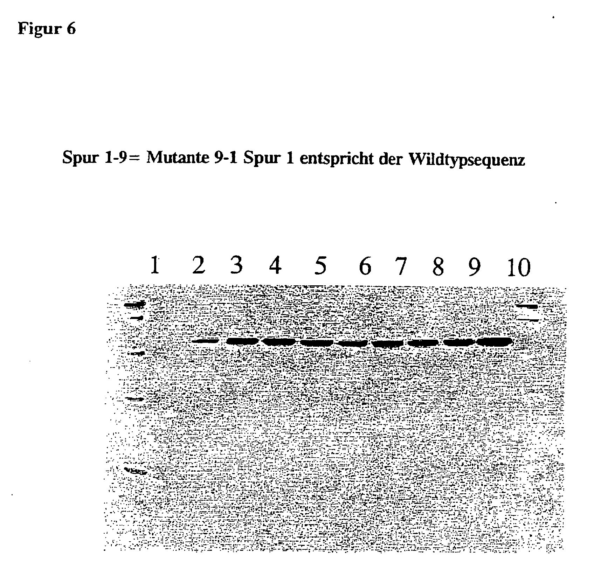

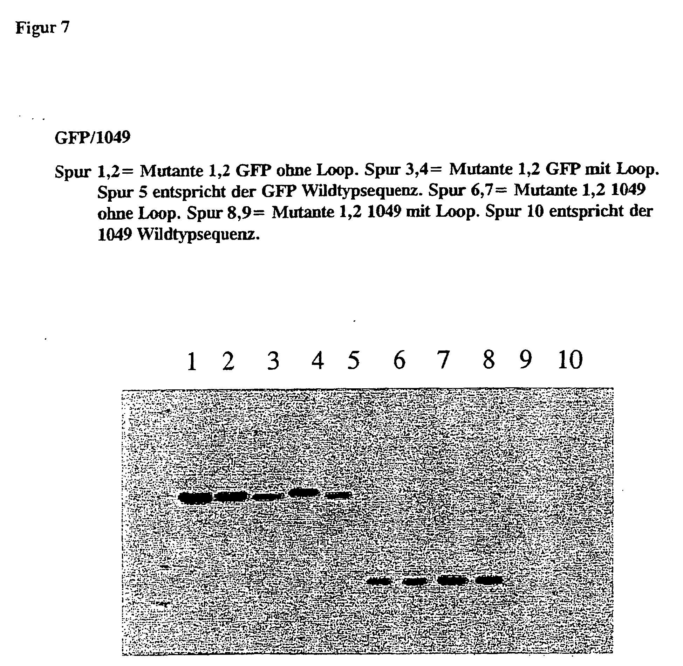

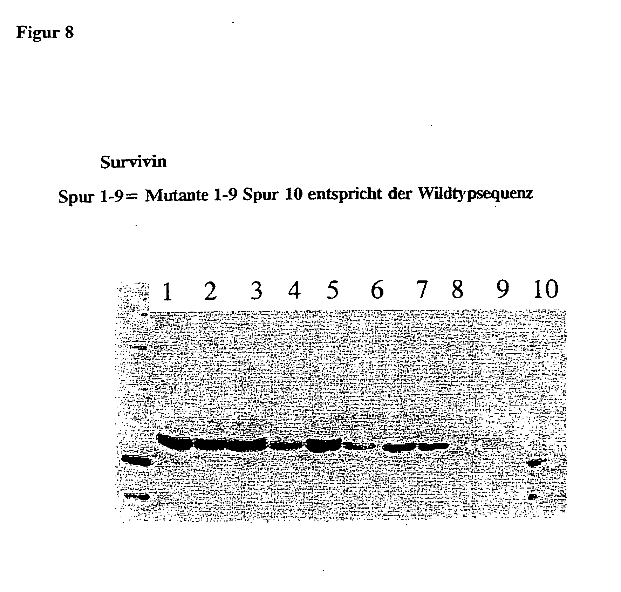

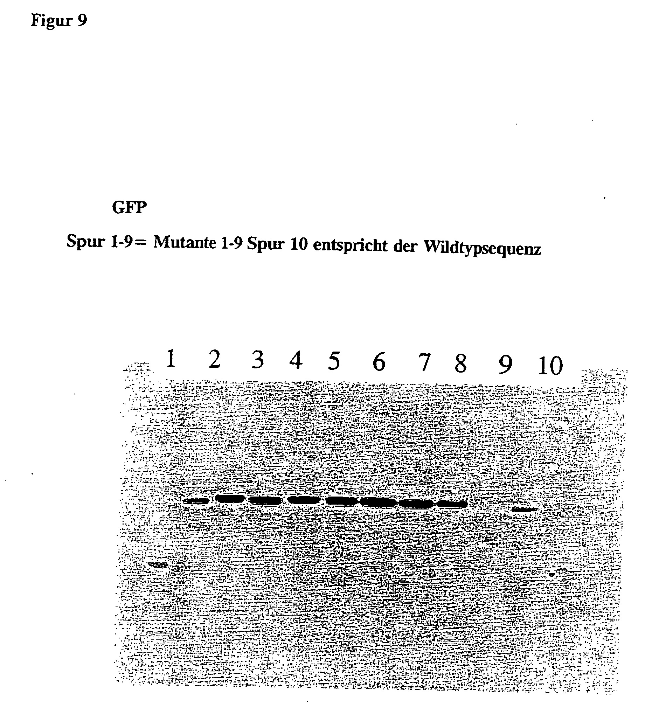

Man kann an den in den Figuren 6 bis 9 gezeigten Expressionen erkennen, dass die mit den Stem-Loop-Strukturen synthetisierten DNA Templates in allen Fällen zu einer Proteinsynthese führten, während beim Wildtypgen keine Proteinsynthese stattfand. Die Mutante 9 mit der Hexa-Histidin Sequenz wird dabei etwas schlechter als die übrigen AT-reichen Sequenzen exprimiert, hat aber den Vorteil, dass man das entstehende Protein über diese Markierung mit sechs Histidinresten an Ni-NTA-Chelat Säulen aufreinigen kann. Auch im Falle des GFP-Gens, einem ohnehin gut exprimierten Gen, führten die Stem-Loop-Konstrukte zu einer Ausbeutesteigerung.

### Beispiel 6: Entfernen der Stem-Loop-Struktur zum Nachweis der Funktion

Um die Wirkung der Stem-Loop-Struktur von der Wirkung der eingeführten AT-reichen Sequenz zu unterscheiden wurden von je zwei der Mutanten eine identische PCR jedoch ohne den Teil des Stem-Loops hergestellt und im direkten Vergleich mit den Stem-Loop-Mutanten exprimiert.

An diesen Beispielen lässt sich deutlich der Einfluss der Stem-Loop-Struktur ersehen. Während beim GFP die AT-reiche Sequenz alleine zu einer gesteigerten Expression führt, bringt die Stem-Loop-Sequenz bei den schwer exprimierbaren Genen den entscheidenden Beitrag.

### Beispiel 7: Modifikation der Stem-Loop-Struktur zur Ermittlung der für die Funktion wichtigen Eigenschaften

Um die Wirkung der GC-Basen innerhalb der Stem-Loop-Struktur zu ermitteln, wurde deren Sequenz durch eine AT-reiche Sequenz mit der gleichen freien Energie wie der des GC-reichen Stem-Loops ersetzt.

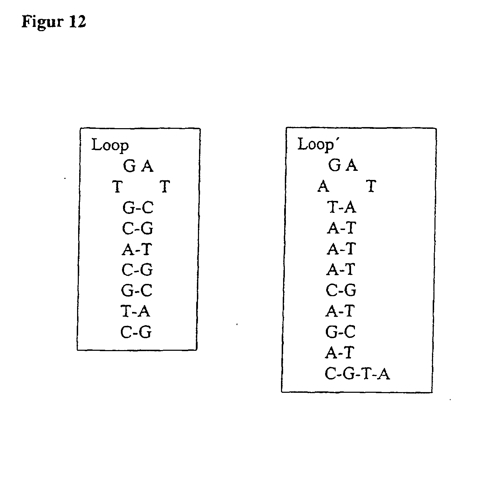

Für die Beispiele Survivin, CIITA und 1049 wurden dafür statt der ursprünglichen Stem-Loop-Sequenz CTG.CAC.GTG.ATC.GTG.CAG mit (G = -9,8 kcal/mol und einer Stammlänge von 7 Basenpaaren ein neuer Stem-Loop (Loop') mit der Sequenz CAG.ACA.AAT.AGA.TAT.TTG.TCT.GTA mit (G = -9,8 kcal/mol und einer Stammlänge von 9 Basenpaaren mit der AT-reichen Sequenz von Mutante 1, kombiniert. Die beiden Strukturen sind in Figur 12 gezeigt.

Man kann erkennen, dass beide Stem-Loop-Varianten die Expression gegenüber den jeweiligen Wildtypgenen deutlich steigern oder erst ermöglichen. Die GC-reichen Stem-Loop-Varianten weisen dabei eine etwas deutlichere Expressionssteigerung auf.

### Beispiel 8: In vivo Protein Expression

Die PCR-Produkte aus Beispiel 5 mit dem Expressionskonstrukt für das Wildtypgen des Cytomegalovirus Capsid Protein 1049 sowie für das Survivin Wildtypgen wurden inpBAD-TOPO (Invitrogen, Carlsbad, USA) Vektoren kloniert. Ebenso wurden jeweils die Expressionskonstrukte für Mutanten des Gens für das Cytomegalovirus Capsid Protein 1049 und das Survivin Gen in diesen Vektor kloniert. Danach wurden die Plasmide in B21 pLyS (Stratagene, Amsterdam, Netherlands) Stämme transformiert und auf auf LB Platten mit 100 µg/ml Carbenicillin und 34 *µ*g/ml Chloramphenicol ausgestrichen. Die Inserts wurden durch Sequenzierung überprüft. Für die *in vivo* Protein Expression wurden je 3 Kolonien isoliert und in 4 ml Medium bei 37 °C für 5 h angezogen. Bei Erreichen einer Zelldichte von 10⁸ Zellen/ml, wurde die Expression durch Zugabe von 1 mM IPTG induziert und weitere 2 h inkubiert. Je 1 ml Zellsuspension davon wurde abzentrifugiert (3 min 14000 rpm) und der Niederschlag in SDS Probenpuffer für 20 min bei 95 °C 1400 rpm auf einem Thermoschüttler erhitzt. Je 10 *µ*l davon wurden auf ein SDS Gel aufgetragen und mit einem Western Blot wie in Beispiel 5 analysiert.

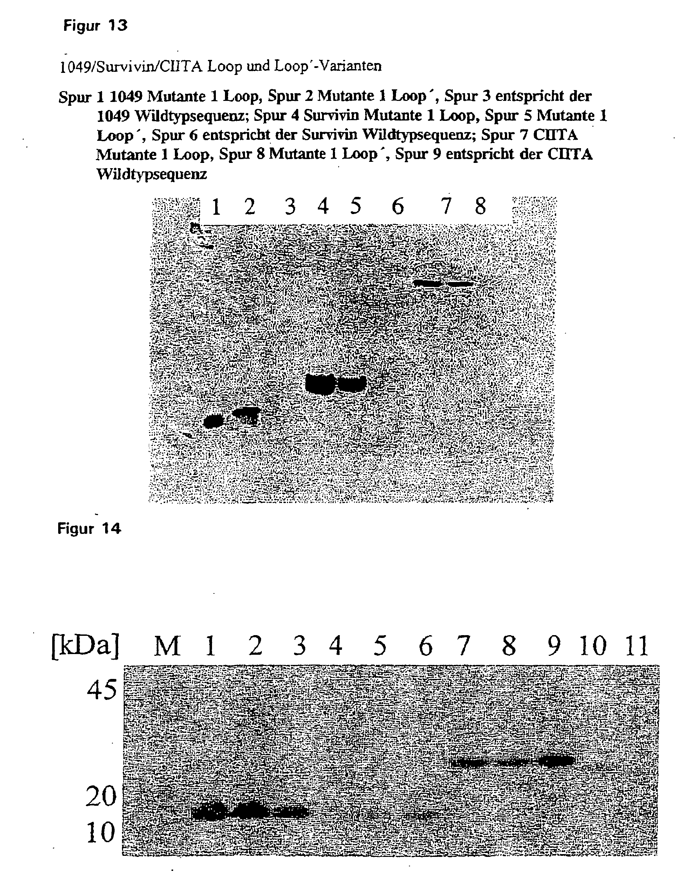

Die Expressionen in Figur 14 zeigen, dass die Stem-Loop-Konstrukte für die beiden untersuchten Gene Cytomegalovirus Capsid Protein 1049 sowie Survivin auch *in vivo* eine wesentliche höhere Expression als die Wildtypgene aufweisen. Dies beweist, dass sich die Ergebnisse der *in vitro* Expression auf die *in vivo* Expression übertragen lassen.

## Claims

1. Method for producing a protein comprising the steps:
(a) providing a nucleic acid sequence coding for the protein in which a heterologous nucleic acid sequence is inserted on the 3' side of the translation start codon in the correct reading frame, said heterologous nucleic acid sequence being selected such that a stem-loop structure is formed on the 3' side of the translation start codon at a distance of 6-30 nucleotides,
(b) providing an expression system suitable for expressing the protein and
(c) introducing the nucleic acid sequence according to (a) into the expression system according to (b) under conditions such that a stem-loop structure is formed wherein the length of the stem is in the range of 4-12 nucleotides.

2. Method as claimed in claim 1 additionally comprising the isolation of the protein.

3. Method as claimed in claim 1 or 2,
**characterized in that**,
the inserted heterologous nucleic acid sequence has a length of up to 201 nucleotides.

4. Method as claimed in claim 3,
**characterized in that**
the inserted heterologous nucleic acid sequence has a length of up to 45 nucleotides.

5. Method as claimed in one of the claims 1 to 4,
**characterized in that**
the stem-loop structure is formed at a distance of 12-21 nucleotides on the 3' side of the start codon.

6. Method as claimed in one of the claims 1 to 5,
**characterized in that**
the region of the heterologous nucleic acid sequence that is on the 5' side of the stem-loop structure does not itself form a secondary structure and cannot form a secondary structure with the 5' untranslated region of the nucleic acid sequence coding for the protein to be produced.

7. Method as claimed in one of the claims 1 to 6,
**characterized in that**
the region of the heterologous nucleic acid sequence that is on the 5' side of the stem-loop structure and on the 3' side of the ATG start codon has a GC content of < 50 %.

8. Method as claimed in one of the claims 1 to 7,
**characterized in that**
an *in vitro* expression system is used.

9. Method as claimed in one of the claims 1 to 8,
**characterized in that**
a prokaryotic *in vitro* expression system is used.

10. Method as claimed in claim 9,
**characterized in that**
the prokaryotic *in vitro* expression system comprises lysates of gram-negative bacteria, in particular of *Escherichia coli* of gram-positive bacteria, in particular of *Bacillus subtilis.*

11. Method as claimed in claim 8,
**characterized in that**
a eukaryotic *in vitro* expression system is used.

12. Method as claimed in claim 11,
**characterized in that**
the eukaryotic *in vitro* expression system comprises lysates of mammalian cells in particular of rabbits, reticulocytes, human tumour cell lines, hamster cell lines or other vertebrate cells, in particular oocytes and eggs of fish and amphibia as well as insect cell lines, yeast cells, algal cells or extracts of plant seedlings.

13. Method as claimed in one of the claims 1 to 7,
**characterized in that**
a prokaryotic *in vivo* expression system is used.

14. Method as claimed in claim 13,
**characterized in that**
a prokaryotic host cell is used as the expression system.

15. Method as claimed in claim 14,
**characterized in that**
a gram-negative prokaryotic host cell, in particular an *E*. *coli* cell or a gram-positive prokaryotic host cell, in particular a *Bacillus subtilis* cell is used.

16. Method as claimed in one of the claims 1 to 7,
**characterized in that**
a eukaryotic host cell is used as an expression system.

17. Method as claimed in claim 16,
**characterized in that**
a yeast cell, an insect cell or a vertebrate cell, in particular an amphibian, fish, bird or mammalian cell is used.

18. Method as claimed in one of the claims 1 to 7,
**characterized in that**
a non-human eukaryotic host organism is used as the expression system.

19. Method as claimed in one of the claims 1 to 18,
**characterized in that**
the nucleic acid sequence coding for the protein is provided by cloning, recombination or/and amplification.

20. Method as claimed in claim 19,
**characterized in that**
the provision comprises a two-step PCR.

21. Method as claimed in one of the claims 1 to 20,
**characterized in that**
the nucleic acid sequence coding for the protein to be produced or/and the heterologous nucleic acid sequence at least partially have a codon usage adapted to the respective expression system.

22. Method as claimed in one of the claims 1 to 21,
**characterized in that**
the heterologous nucleic acid sequence contains a section coding for a purification domain or/and a section coding for a proteinase recognition domain.

23. Reagent for producing a protein comprising
(a) a nucleic acid sequence that is heterologous to the nucleic acid sequence coding for the protein which can be inserted into the protein-coding nucleic acid sequence in the correct reading frame and which can form a stem-loop structure at a distance of 6-30 nucleotides on the 3' side of the translation start codon, and
(b) an expression system that is suitable for producing the protein.

## Patentansprüche

1. Verfahren zur Herstellung eines Proteins, umfassend die Schritte:
(a) Bereitstellen einer für das Protein kodierenden Nukleinsäuresequenz, wobei 3'-seitig des Translations-Startcodons eine heterologe Nukleinsäuresequenz im korrekten Leseraster eingefügt wird, die so gewählt wird, dass in einem Abstand von 6-30 Nukleotiden 3'-seitig des Translations-Startcodons eine Stem-Loop-Struktur ausgebildet wird,
(b) Bereitstellen eines zur Expression des Proteins geeigneten Expressionssystems und
(c) Einbringen der Nukleinsäuresequenz gemäß (a) in das Expressionssystem gemäß (b) unter Bedingungen, dass eine Stem-Loop-Struktur ausgebildet wird, worin die Länge des Stem im Bereich von 4-12 Nukleotiden ist.

2. Verfahren nach Anspruch 1, weiterhin umfassend das Gewinnen des Proteins.

3. Verfahren nach Anspruch 1 oder 2,
**dadurch gekennzeichnet,**
**dass** die eingefügte heterologe Nukleinsäuresequenz eine Länge bis zu 201 Nukleotiden aufweist.

4. Verfahren nach Anspruch 3,
**dadurch gekennzeichnet,**
**dass** die eingefügte heterologe Nukleinsäuresequenz eine Länge bis zu 45 Nukleotiden aufweist.

5. Verfahren nach einem der Ansprüche 1 bis 4,
**dadurch gekennzeichnet,**
**dass** die Stem-Loop-Struktur in einem Abstand von 12-21 Nukleotiden 3'-seitig des Startcodons ausgebildet wird.

6. Verfahren nach einem der Ansprüche 1 bis 5,
**dadurch gekennzeichnet,**
**dass** der Bereich der heterologen Nukleinsäuresequenz, der 5'-seitig der Stem-Loop-Struktur liegt, selbst keine Sekundärstruktur ausbildet und keine Sekundärstruktur mit der 5'-untranslatierten Region der für das herzustellende Protein kodierenden Nukleinsäuresequenz eingehen kann.

7. Verfahren nach einem der Ansprüche 1 bis 6,
**dadurch gekennzeichnet,**
**dass** der Bereich der heterologen Nukleinsäuresequenz, der 5'-seitig der Stem-Loop-Struktur und 3'-seitig des ATG-Startcodons liegt, einen GC-Gehalt von <50 % aufweist.

8. Verfahren nach einem der Ansprüche 1 bis 7,
**dadurch gekennzeichnet,**
**dass** man ein *in vitro* Expressionssystem verwendet.

9. Verfahren nach Anspruch 8,
**dadurch gekennzeichnet,**
**dass** man ein prokaryontisches *in vitro* Expressionssystem verwendet.

10. Verfahren nach Anspruch 9,
**dadurch gekennzeichnet,**
**dass** das prokaryontische *in vitro* Expressionssystem Lysate von gram-negativen Bakterien, insbesondere von *Escherichia coli,* oder gram-positiven Bakterien, insbesondere *Bacillus subtilis,* enthalten.

11. Verfahren nach Anspruch 8,
**dadurch gekennzeichnet,**
**dass** man ein eukaryontisches *in vitro* Expressionssystem verwendet.

12. Verfahren nach Anspruch 11,
**dadurch gekennzeichnet,**
**dass** das eukaryontische *in vitro* Expressionssystem Lysate von Säugerzelien, insbesondere von Kaninchen, Reticulocyten, humanen Tumorzelllinien, Hamsterzelllinien, oder anderen Wirbeltierzellen, insbesondere Oozyten und Eiern von Fischen und Amphibien, sowie Insektenzelllinien, Hefezellen, Algenzellen oder Extrakte aus Pflanzenkeimen enthält.

13. Verfahren nach einem der Ansprüche 1 bis 7
**dadurch gekennzeichnet,**
**dass** man ein prokaryontisches *in vivo* Expressionssystem verwendet.

14. Verfahren nach Anspruch 13,
**dadurch gekennzeichnet,**
**dass** man eine prokaryontische Wirtszelle als Expressionssystem verwendet.

15. Verfahren nach Anspruch 14,
**dadurch gekennzeichnet,**
**dass** man eine gram-negative prokaryontische Wirtszelle, insbesondere eine E.coli Zelle, oder eine gram-positive prokaryontische Wirtszelle, insbesondere eine *Bacillus subtilis* Zelle, verwendet.

16. Verfahren nach einem der Ansprüche 1 bis 7,
**dadurch gekennzeichnet,**
**dass** man eine eukaryontische Wirtszelle als Expressionssystem verwendet.

17. Verfahren nach Anspruch 16,
**dadurch gekennzeichnet,**
**dass** man eine Hefezelle, eine Insektenzelle oder eine Wirbeltierzelle, insbesondere eine Amphibien-, Fische-, Vogel- oder Säugerzelle, verwendet.

18. Verfahren nach einem der Ansprüche 1 bis 7,
**dadurch gekennzeichnet,**
**dass** man einen nicht-humanen eukaryontischen Wirtsorganismus als Expressionssystem verwendet.

19. Verfahren nach einem der Ansprüche 1 bis 18,
**dadurch gekennzeichnet,**
**dass** das Bereitstellen der für das Protein kodierenden Nukleinsäuresequenz durch Klonierung, Rekombination oder/und Amplifikation erfolgt.

20. Verfahren nach Anspruch 19,
**dadurch gekennzeichnet,**
**dass** das Bereitstellen eine Zweistufen=PCR umfasst.

21. Verfahren nach einem der Ansprüche 1 bis 20,
**dadurch gekennzeichnet,**
**dass** die für das herzustellende Protein kodierende Nukleinsäuresequenz oder/und die heterologe Nukleinsäuresequenz zumindest teilweise eine an das jeweiligen Expressionssystem angepasste Codon-Nutzung aufweisen.

22. Verfahren nach einem der Ansprüche 1 bis 21,
**dadurch gekennzeichnet,**
**dass** die heterologe Nukleinsäuresequenz einen für eine Aufreinigungsdomäne oder/und einen für eine Proteinase-Erkennungsdomäne kodierenden Abschnitt enthält.

23. Reagenz zur Herstellung eines Proteins, umfassend
(a) eine zu der für das Protein kodierenden Nukleinsäuresequenz heterologe Nukleinsäuresequenz, die im korrekten Leseraster in die Protein-kodierende Nukleinsäuresequenz eingefügt werden kann, und die in einem Abstand von 6-30 Nukleotiden 3'-seitig des Translations-Startcodons eine Stem-Loop-Struktur ausbilden kann, und
(b) ein zur Herstellung des Proteins geeignetes Expressionssystem.

## Revendications

1. Procédé de préparation d'une protéine, comprenant les étapes:
(a) préparation d'une séquence d'acide nucléique codant pour la protéine, avec insertion du côté 3' du codon d'initiation de la traduction, dans le cadre de lecture correct, d'une séquence d'acide nucléique hétérologue qui est choisie de façon qu'il se forme une structure en tige et boucle à une distance de 6-30 nucléotides du côté 3' du codon d'initiation de la traduction,
(b) préparation d'un système d'expression approprié pour l'expression de la protéine, et
(c) introduction de la séquence d'acide nucléique selon (a) dans le système d'expression selon (b) dans des conditions dans lesquelles il se forme une structure en tige et boucle, dans laquelle la longueur de la tige se situe dans le domaine de 4-12 nucléotides.

2. Procédé selon la revendication 1, comprenant en outre la récupération de la protéine.

3. Procédé selon la revendication 1 ou 2, **caractérisé en ce que** la séquence d'acide nucléique hétérologue insérée a une longueur allant jusqu'à 201 nucléotides.

4. Procédé selon la revendication 3, **caractérisé en ce que** le séquence d'acide nucléique hétérologue insérée a une longueur allant jusqu'à 45 nucléotides.

5. Procédé selon l'une des revendications 1 à 4, **caractérisé en ce que** la structure en tige et boucle est formée à une distance de 12-21 nucléotides du côté 3' du codon d'initiation.

6. Procédé selon l'une des revendications 1 à 5, **caractérisé en ce que** le domaine de la séquence d'acide nucléique hétérologue qui se situe du côté 5' de la structure en tige et boucle ne forme pas lui-même de structure secondaire et ne peut pas engendrer de structure secondaire avec la région non traduite en 5' de la séquence d'acide nucléique codant pour la protéine à préparer.

7. Procédé selon l'une des revendications 1 à 6, **caractérisé en ce que** le domaine de la séquence d'acide nucléique hétérologue qui se situe du côté 5' de la structure en tige et boucle et du côté 3' du codon d'initiation ATG présente une teneur en GC inférieure à 50 %.

8. Procédé selon l'une des revendications 1 à 7, **caractérisé en ce que** l'on utilise un système d'expression *in vitro.*

9. Procédé selon la revendication 8, **caractérisé en ce que** l'on utilise un système d'expression *in vitro* procaryote.

10. Procédé selon la revendication 9, **caractérisé en ce que** le système d'expression *in vitro* procaryote contient des lysats de bactéries gram-négatives, en particulier *d'Escherichia coli,* ou de bactéries gram-négatives, en particulier de *Bacillus subtilis.*

11. Procédé selon la revendication 8, **caractérisé en ce que** l'on utilise un système d'expression *in vitro* eucaryote.

12. Procédé selon la revendication 11, **caractérisé en ce que** le système d'expression *in vitro* eucaryote contient des lysats de cellules de mammifère, en particulier de lapin, de réticulocytes, de lignées cellulaires tumorales humaines, de lignées cellulaires de hamster, ou d'autres cellules de vertébrés, en particulier d'ovocytes et d'oeufs de poissons et d'amphibiens, ainsi que de lignées cellulaires d'insectes, de cellules de levure, de cellules d'algues ou des extraits de germes de plantes.

13. Procédé selon l'une des revendications 1 à 7, **caractérisé en ce que** l'on utilise un système d'expression *in vivo* procaryote.

14. Procédé selon la revendication 13, **caractérisé en ce que** l'on utilise une cellule hôte procaryote comme système d'expression.

15. Procédé selon la revendication 14, **caractérisé en ce que** l'on utilise une cellule hôte procaryote gram-négative, en particulier une cellule d'*E*. *coli,* ou une cellule hôte procaryote gram-positive, en particulier une cellule de *Bacillus subtilis.*

16. Procédé selon l'une des revendications 1 à 7, **caractérisé en ce que** l'on utilise une cellule hôte eucaryote comme système d'expression.

17. Procédé selon la revendication 16, **caractérisé en ce que** l'on utilise une cellule de levure, une cellule d'insecte ou une cellule de vertébré, en particulier une cellule d'amphibien, de poisson, d'oiseau ou de mammifère.

18. Procédé selon l'une des revendications 1 à 7, **caractérisé en ce que** l'on utilise un organisme hôte eucaryote non humain comme système d'expression.

19. Procédé selon l'une des revendications 1 à 18, **caractérisé en ce que** la préparation de la séquence d'acide nucléique codant pour la protéine s'effectue par clonage, recombinaison et/ou amplification.

20. Procédé selon la revendication 19, **caractérisé en ce que** la préparation comprend une PCR en deux étapes.

21. Procédé selon l'une des revendications 1 à 20, **caractérisé en ce que** la séquence d'acide nucléique codant pour la protéine à préparer et/ou la séquence d'acide nucléique hétérologue présentent au moins en partie une utilisation des codons adaptée au système d'expression respectif.

22. Procédé selon l'une des revendications 1 à 21, **caractérisé en ce que** la séquence d'acide nucléique hétérologue contient un fragment codant pour un - domaine de purification et/ou pour un domaine de reconnaissance de protéinase.

23. Réactif pour la préparation d'une protéine, comprenant:
(a) une séquence d'acide nucléique hétérologue à la séquence d'acide nucléique codant pour la protéine, qui peut être insérée dans le cadre de lecture correct dans la séquence d'acide nucléique codant pour la protéine, et qui peut former une structure en tige et boucle à une distance de 6-30 nucléotides du côté 3' du codon d'initiation de la traduction, et
(b) un système d'expression approprié pour la préparation de la protéine.
